# EUROPEAN PATENT SPECIFICATION

(11) **EP 2 873 424 B1**
(45) Date of publication and mention of the grant of the patent: **18.09.2019**
(21) Application number: 13794652.1
(22) Date of filing: 23.05.2013
(51) Int. Cl.: A61K 39/395, A61K 48/00, A61K 31/7105, A61P 25/28, G01N 33/50, A61K 31/4545, A61K 31/506, A61K 31/7088, C07K 14/705, C07K 16/18, C07K 16/40

(54) **TAU PROTEIN MEDIATED NEURODEGENERATIVE DISEASE THERAPEUTIC AGENT**
TAU-PROTEIN-VERMITTELTER THERAPEUTISCHER WIRKSTOFF FÜR NEURODEGENERATIVE ERKRANKUNGEN
AGENT THÉRAPEUTIQUE DESTINÉ À UNE MALADIE NEURODÉGÉNÉRATIVE MÉDIÉE PAR LA PROTÉINE TAU

(30) Priority: 24.05.2012 KR 20120055163
(43) Date of publication of application: 20.05.2015
(73) Proprietor: Seoul National University R&DB Foundation, Seoul 151-015 (KR)
(72) Inventor: JUNG, Yong Keun, Seoul 151-055 (KR); CHOI, Hyun Woo, Seoul 08824 (KR)
(74) Representative: Dehns
(86) International application number: PCT/KR2013/004537
(87) International publication number: WO 2013/176503

(56) References cited:
- WO-A1-2007/088400
- WO-A2-2005/123048
- WO-A2-2007/023310
- US-A1- 2002 034 768
- US-A1- 2011 294 144
- BILSLAND J.G. ET AL.: "Behavioral and neurochemical alterations in mice deficient in anaplastic lymphoma kinase suggest therapeutic potential for psychiatric indications", NEUROPSYCHOPHARMACOL., vol. 33, 9 May 2007 (2007-05-09), pages 685-700, XP55180524,
- LI Y. ET AL.: "Evaluation of EML4-ALK fusion proteins in non-small cell lung cancer using small molecule inhibitors", NEOPLASIA, vol. 13, no. 1, January 2011 (2011-01), pages 1-11, XP002672742,
- CHIARLE R. ET AL.: "The anaplastic lymphoma kinase in the pathogenesis of cancer", NATURE REV., vol. 8, no. 1, January 2008 (2008-01), pages 11-23, XP002514871,
- WEISS ET AL.: 'Anaplastic Lymphoma Kinase and Leukocyte Tyrosine Kinase: Functions and genetic interactions in learning, memory and adult neurogenesis' PHARMACOLOGY, BIOCHEMISTRY AND BEHAVIOR vol. 100, 2012, pages 566 - 574, XP028340891
- BILSLAND ET AL.: 'Behavioral and Neurochemical Alterations in Mice Deficient in Anaplastic Lymphoma Kinase Suggest Therapeutic Potential for Psychiatric Indications' NEUROPSYCHOPHARMACOLOGY vol. 33, 2008, pages 685 - 700, XP055180524

<110> SEOUL NATIONAL UNIVERSITY R&DB FOUNDATION
<120> Therapeutics for the treatment of neurodegenerative diseases mediated by Tau proteins
<130> PT13-0196
<150> KR 2012/0055163
   <151> 2012-05-24
<160> 2
<170> KopatentIn 1.71
<210> 1
   <211> 19
   <212> DNA
   <213> Artificial Sequence
<220>
   <223> pCDK5 shRNA #1
<400> 1
   tgaccaagct gccagacta 19
<210> 2
   <211> 19
   <212> DNA
   <213> Artificial Sequence
<220>
   <223> pCDK5 shRNA #2
<400> 2
   actccacgtc catcgacat 19

## Description

### TECHNICAL FIELD

The present invention relates to a pharmaceutical composition for treating a degenerative neuronal disease, and more particularly, to a pharmaceutical composition for treating a degenerative neuronal disease mediated by tau protein.

### BACKGROUND

It has been known that tau consists of four parts, which are an N-terminal protrusion part, a proline-rich domain, a microtubule-binding domain, and c-terminal (see Mandelkow et al., Acta. Neuropathol., 103, 26-35, 1996), and a degenerative neuronal disease such as tauopathy results from abnormally hyperphosphorylated or modified tau in neuronal cells of a central nervous system. Typical tauopathy includes Alzheimer's disease, Pick's disease, frontotemporal dementia and parkinsonism disease linked to chromosome 17 (FTDP-17) *etc.* (see Lee et al., Annu. Rev. Neurosci., 24: 1121-1159, 2001; Bergeron et al., J. Neuropathol. Exp. Neurol., 56: 726-734, 1997; Bugiani et al., J. Neuropathol. Exp. Neurol., 58: 667-677, 1999; Delacourte et al., Ann. Neurol., 43: 193-204, 1998; Ittner and Gotz, Nat. Rev. Neurosci., 12: 65-72, 2011).

For example, Korean Patent Publication No. 2009-0043251 discloses a therapeutic agent for a degenerative neuronal disease including, as an active ingredient, an inhibitor of target gene expression or activity; and US6057117 provides a GSK3-specific inhibitor for treating an active GSK3-mediated disease including non-insulin dependent diabetes mellitus (NIDDM) and Alzheimer's disease.WO 2007/023310 A2 provides a method for increasing neurogenesis in the brain by administering an inhibitor of anaplastic lymphoma kinase (ALK), the treatment of cognitive dysfunction and depression disorders by administering an inhibitor of ALK, an assay for determining inhibitors of ALK, and inhibitors of ALK.

### DETAILED DESCRIPTION OF INVENTION

### TECHNICAL PROBLEM

However, among typical therapeutic agents for degenerative neuronal diseases, it has been not well developed a therapeutic agent for a neuronal disease targeting tau protein. Further, the mechanism of causing tau-mediated degenerative neuronal diseases has not been clearly determined yet.

The present invention provides a novel target for treating a degenerative neuronal disease by determining a specific signaling mechanism which mediates phosphorylation, aggregation, and neurotoxicity of tau protein, in order to provide a therapeutic agent for a tau protein-mediated degenerative neuronal disease. The present invention also provides a pharmaceutical composition for screening a therapeutic agent using a signaling mechanism of the tau protein-mediated degenerative neuronal disease. However, these solutions are provided for illustrative purpose only, and thus the scope of the present invention is not limited thereto.

### PROBLEM SOLUTION

According to an aspect of the present invention, provided is a pharmaceutical composition for treating a degenerative neuronal disease in a subject suffering from a degenerative neuronal disease caused by hyper-phosphorylation or aggregation of tau protein or neuronal cell death, comprising a therapeutically effective amount of an inhibitor of the dimerization, the activation or the expression of anaplastic lymphoma kinase (ALK).

According to the pharmaceutical composition, the inhibitor may be an antagonizing antibody to ALK or a functional derivative thereof, an ALK-specific inhibiting compound, an anti-sense nucleotide, siRNA, shRNA, or miRNA against an ALK gene.

According to the pharmaceutical composition, the inhibitor may be an ALK kinase activity inhibitor. For example, the ALK kinase activity inhibitor may be NVP-TAE684, PF-2341066, crizotinib (trade name Xalkori), AP26113, or LDK378, but not limited thereto.

According to the pharmaceutical composition, the degenerative neuronal disease may be Alzheimer's disease or frontotemporal lobar degeneration.

According to an example of the present invention, in the pharmaceutical composition, a compound which is known to inhibit ALK activity may be used as the ALK-specific inhibiting compound such as the 2,4-pyrimidine derivative disclosed in Korean Registered Patent No. 0904570, the pyrazoloimidazole-based compound disclosed in Korean Registered Patent No. 1083421, the 1,6-substituted indole compound disclosed in Korean Registered Patent No. 1116756, the 2,4,7-substituted thieno[3,2,-d] pyrimidine compound disclosed in Korean Registered Patent No. 1094446, the bicyclic heteroaryl derivative disclosed in Korean Patent Publication No. 2011-0088960, the furo[3,2,-c]pyridine and thieno[3,2-c]pyridine compounds disclosed in Korean Patent Publication No. 2011-0014971, and the pyrazole-substitutied aminoheteroaryl compound disclosed in WO2006/021881A2.

The pharmaceutical composition may be administrated by an oral administration or a parenteral administration. In the case of parenteral administration, administration may be performed through an intraperitoneal injection, an intrarectal injection, a subcutaneous injection, an intravenous injection, an intramuscular injection, an intrauterine epidural injection, an intracranial injection, an intracerebroventricular injection, an intracerebrospinal injection, an intrathecal injection, an intracerebrovascular injection or an intrathoracic injection in a form of a general pharmaceutical product.

According to an example of the present invention, in the pharmaceutical composition, the ALK inhibitor may be administered in a form of a pharmaceutical composition further including a non-active ingredient including a pharmaceutically acceptable carrier. As used herein, the term "pharmaceutically acceptable carrier" specifically refers to an ingredient of a pharmaceutical composition expect an active ingredient. Examples of a pharmaceutically acceptable carrier include a binding agent, a disintegrating agent, a diluting agent, a filler, a lubricating agent, a solubilizing agent or an emulsifying agent, and a salt.

For substantial use, the pharmaceutical composition according to an example of the present invention may be combined with a pharmaceutical carrier by the typical pharmaceutical preparation technique. The carrier may have a wide range of forms depending on a preferred preparation for oral or parenteral administration including intravenous administration for example.

In addition to, the pharmaceutical composition according to an example of the present invention may be administered in a dose of 0.1 mg/kg to 1 g/kg, and more preferably in a dose of 0.1 mg/kg to 500 mg/kg. The administration dose may be adequately adjusted depending on age, sex, and condition of patients.

In another aspect of the present invention, provided is a method for treating a degenerative disease caused by the phosphorylation or aggregation of tau protein administrating therapeutically effective amount of the pharmaceutical composition according to an example of the present invention to a subject suffering the degenerative disease.

According to the method, the degenerative neuronal disease may be Alzheimer's disease or frontotemporal lobar degeneration.

In an aspect of the present invention, provided is a method for screening a drug for treating a degenerative disease caused by the phosphorylation or aggregation of tau protein or apoptosis of neuronal cells comprising: treating a test compound to a reaction solution including ALK, ATP, and a substrate of ALK; measuring a change in a phosphorylation level of the substrate of ALK; and selecting a test compound which significantly inhibits phosphorylation of the substrate of ALK compared to a that of a negative control untreated with the test compound;

According to the method, the degenerative neuronal disease may be Alzheimer's disease or frontotemporal lobar degeneration.

According to the method, the substrate of ALK may be Ras/MEK/ERK, PI3K/AKT, JAK3/STAT3 signaling proteins.

In an aspect of the present invention, provided is a method for screening a drug for treating a degenerative disease caused by the phosphorylation or aggregation of tau protein or apoptosis of neuronal cells comprising: examining the phosphorylation of substrate of ALK or tau protein, or the aggregation of the tau protein in cells treated with the test compound; and selecting a test compound which inhibits phosphorylation of the substrate of ALK, or the tau protein, or the aggregation of the tau protein compared to the negative control untreated with the test compound.

According to the method, the degenerative neuronal disease may be Alzheimer's disease or frontotemporal lobar degeneration.

According to the method, the substrate of ALK may be Ras/MEK/ERK, PI3K/AKT, JAK3/STAT3 signaling proteins.

In an aspect of the present invention, provided is a method for screening a drug for treating a degenerative disease caused by the phosphorylation or aggregation of tau protein or apoptosis of neuronal cells comprising: treating a test compound to reaction solution including ALK, ATP, and a substrate of ALK; and selecting a test compound which inhibits an interaction between ALK and the substrate of ALK compared to the negative control untreated with the test compound;

According to the method, the degenerative neuronal disease may be Alzheimer's disease or frontotemporal lobar degeneration.

According to the method, the substrate of ALK may be Ras/MEK/ERK, PI3K/AKT, JAK3/STAT3 signaling proteins.

According to the method, the interaction between ALK and a substrate or a ligand may be examined by using a surface plasmon resonance (SPR) assay, a fluorescence resonance energy transfer (FRET) system, an immunofluorescence staining, an immunoprecipitation (IPP), a GST-Pull down assay, a yeast two-hybrid system (Y2H), a bimolecular fluorescence complementation (BiFC) method, and a tandem affinity purification (TAP)-tag method, *etc.*

In an aspect of the present invention, provided is a method for screening a drug for treating a degenerative disease caused by the phosphorylation or aggregation of tau protein or apoptosis of neuronal cells comprising: treating cells expressing ALK and a substrate of ALK with a test compound; and selecting a test compound which inhibits an interaction between ALK and the substrate of ALK compared to the negative control untreated with the test compound.

According to the method, the degenerative neuronal disease may be Alzheimer's disease or frontotemporal lobar degeneration.

According to the method, the substrate of ALK may be Ras/MEK/ERK, PI3K/AKT, JAK3/STAT3 signaling proteins.

According to the method, the interaction between ALK with a substrate or a ligand may be examined by using a surface plasmon resonance (SPR) assay, a fluorescence resonance energy transfer (FRET) system, an immunofluorescence staining, an immunoprecipitation (IPP), a GST-Pull down assay, a yeast two-hybrid system (Y2H), a bimolecular fluorescence complementation (BiFC) method, and a tandem affinity purification (TAP)-tag method, *etc.*

In an aspect of the present invention, provided is a method for screening a drug for treating a degenerative disease caused by the phosphorylation or aggregation of tau protein or apoptosis of neuronal cells comprising: treating cells expressing ALK with a test compound and a ligand of ALK; and selecting a test compound which inhibits an interaction between ALK and the ligand of ALK compared to the negative control untreated with the test compound.

According to the method, the degenerative neuronal disease may be Alzheimer's disease or frontotemporal lobar degeneration.

According to the method, the ALK ligand may be pleiotrophin (PTN), midkine (MK) or Jelly belly (Jeb).

According to the method, the interaction between ALK with a substrate or a ligand may be examined by using a surface plasmon resonance (SPR) assay, a fluorescence resonance energy transfer (FRET) system, an immunofluorescence staining, an immunoprecipitation (IPP), a GST-Pull down assay, a yeast two-hybrid system (Y2H), a bimolecular fluorescence complementation (BiFC) method, and a tandem affinity purification (TAP)-tag method, *etc.*

In an aspect of the present invention, provided is a method for screening a drug for treating a degenerative disease caused by the phosphorylation or aggregation of tau protein or apoptosis of neuronal cells comprising: treating cells expressing ALK with a test compound and a ligand of ALK; and selecting a test compound which inhibits an interaction between ALK and the ligand of ALK compared to the negative control untreated with the test compound.

According to the method, the degenerative neuronal disease may be Alzheimer's disease or frontotemporal lobar degeneration.

According to the method, the substrate of ALK may be Ras/MEK/ERK, PI3K/AKT, JAK3/STAT3 signaling proteins.

According to the method, the ALK ligand may be pleiotrophin (PTN), midkine (MK) or Jelly belly (Jeb).

According to the method, the interaction between ALK with a substrate or a ligand may be examined by using a surface plasmon resonance (SPR) assay, a fluorescence resonance energy transfer (FRET) system, an immunofluorescence staining, an immunoprecipitation (IPP), a GST-Pull down assay, a yeast two-hybrid system (Y2H), a bimolecular fluorescence complementation (BiFC) method, and a tandem affinity purification (TAP)-tag method, *etc.*

In an aspect of the present invention, provided is a method for screening a drug for treating a degenerative disease caused by the phosphorylation or aggregation of tau protein or apoptosis of neuronal cells comprising: treating cells expressing ALK with a test compound; measuring a change in a phosphorylation level of cyclin-dependent kinase-5 (CDK-5) or extracellular signal-regulated kinase (ERK) in cells treated with the test compound; and selecting a test compound which significantly inhibits phosphorylation of CDK5 or ERK compared to the negative control untreated with the test compound.

According to the method, the degenerative neuronal disease may be Alzheimer's disease or frontotemporal lobar degeneration.

In an aspect of the present invention, provided is a method for screening a drug for treating a degenerative disease caused by the phosphorylation or aggregation of tau protein or apoptosis of neuronal cells comprising: treating a transformed fruitfly expressing ALK and tau (Tau/dALK) with a test compound; observing eye phenotype or neuronal degeneration by measuring a shape of an ommatidium or a change in a retinal thickness of the transformed fruitfly treated with the test compound; and selecting a test compound which significantly inhibits a change in a ommatidium shape or disruption of a retina compared to the negative control untreated with the test compound.

According to the method, the degenerative neuronal disease may be Alzheimer's disease or frontotemporal lobar degeneration.

In an aspect of the present invention, provided is a method for screening a drug for treating a degenerative disease caused by the phosphorylation or aggregation of tau protein or apoptosis of neuronal cells comprising: treating ALK protein with a test compound; observing whether the ALK protein forms a dimer or not; and selecting a test compound which inhibits the dimer formation of ALK protein.

According to the method, the degenerative neuronal disease may be Alzheimer's disease or frontotemporal lobar degeneration.

### EFFECT OF THE INVENTION

In accordance with an example of the present invention made as above described, a symptom of a patient having a degenerative neuronal disease, e.g., decline in cognition and memory, may be alleviated by inhibiting ALK(anaplastic lymphoma kinase) to inhibit phosphorylation, and aggregation of tau protein and neuronal cell death observed in a tau-mediated degenerative neuronal diseases.

### BRIEF DESCRIPTION OF THE DRAWINGS

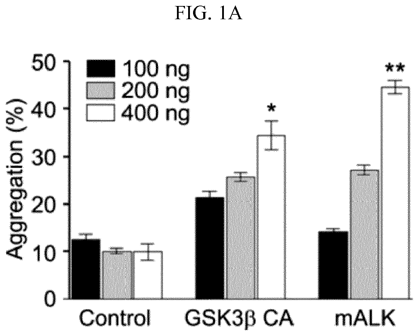

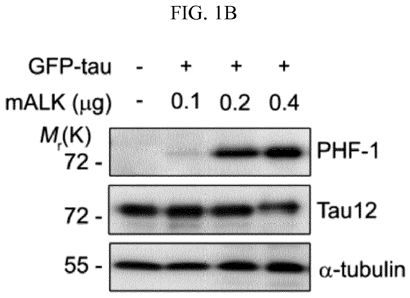

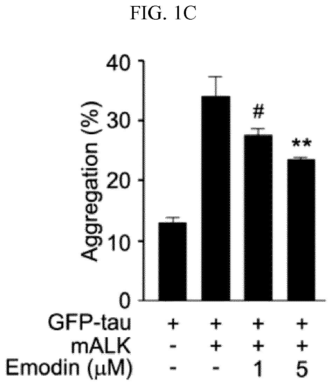

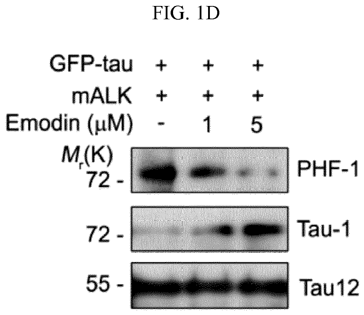

FIG. 1A is a graph showing a result of counting aggregated HT22 cells through a fluorescence microscopy when HT22 cells were cotransfected with pGFP-tau and one of pcDNA, pGSK3*β* CA, and pmALK; FIG. 1B shows a western blot result of cells observed in FIG. 1A examining that tau phosphorylation is increased as an amount of treated pmALK increases; FIG. 1C is a graph showing that the number of aggregated HT22 cells, which is reduced when pGFP-tau and pmALK are cotransfected and then emodin treatment is followed, is counted through a fluorescence microscopy; FIG. 1D shows a western blot result to examine a change in a phosphorylation level of tau depending on an increment in an amount of treated emodin in cells observed in FIG. 1C;

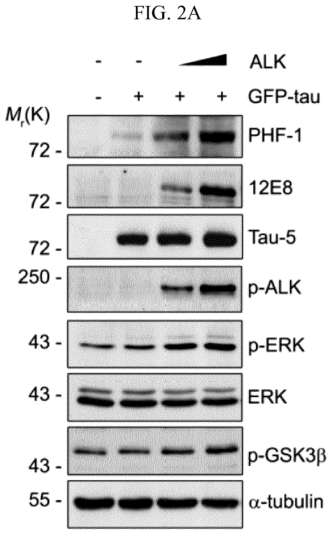

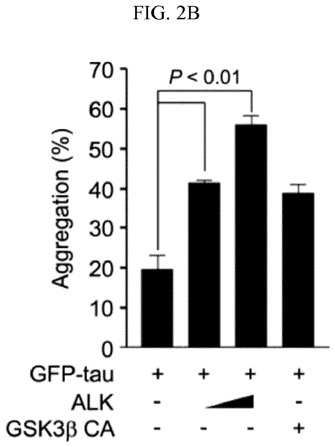

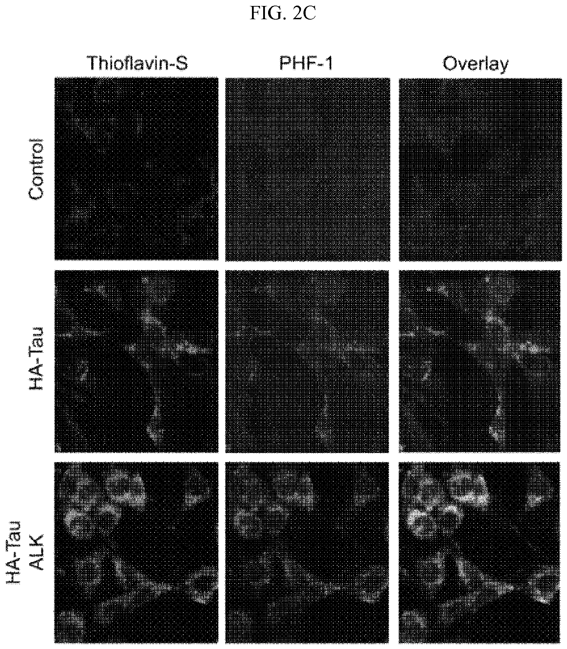

FIG. 2A shows a western blot result examining that phosphorylation of HT22 cells is increased due to cotransfection of pGFP-tau and pALK; FIG. 2B is a graph showing that the number of aggregated HT22 cells, which is increased as an introduced amount of pALK increases when HT22 cells are cotransfected with pGFP-tau and pALK, is counted through fluorescent microscopy; FIG. 2C are fluorescence microscopy images showing that phosphorylation and aggregation of HT22 cells are increased by cotransfection of pHA-Tau and pALK;

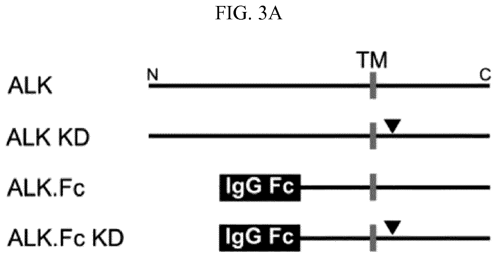

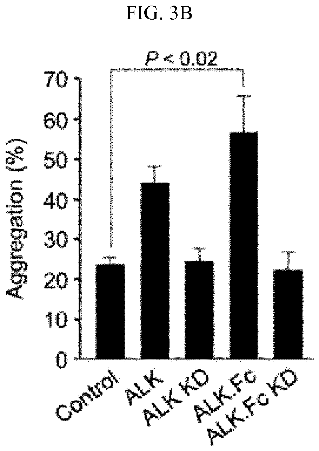

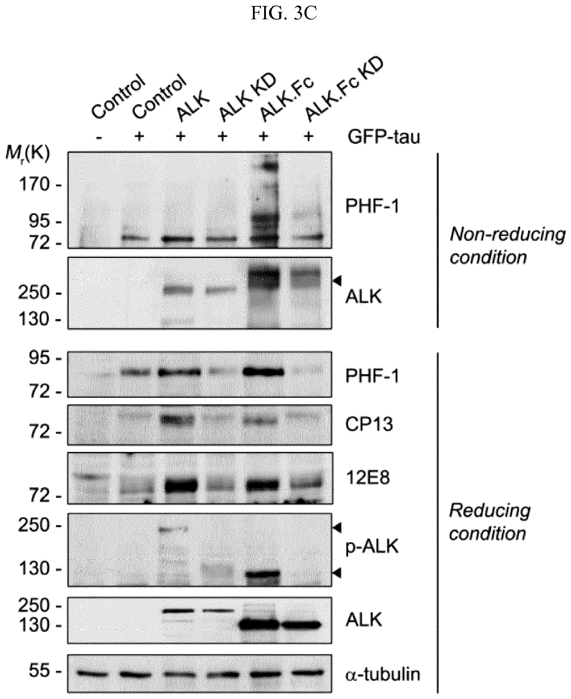

FIG. 3A is a schematic diagram illustrating wild-type human anaplastic lymphoma kinase (ALK) and ALK variant constructs; FIG. 3B is a graph examining that aggregated HT22 cells are reduced when HT22 cells were cotransfected with pGFP-tau and one of ALK constructs (i.e., pALK, pALK KD, pALK.Fc, and pALK.Fc KD) thereby inactivating kinase of ALK; FIG. 3C shows a western blot result of cells observed in FIG. 3B;

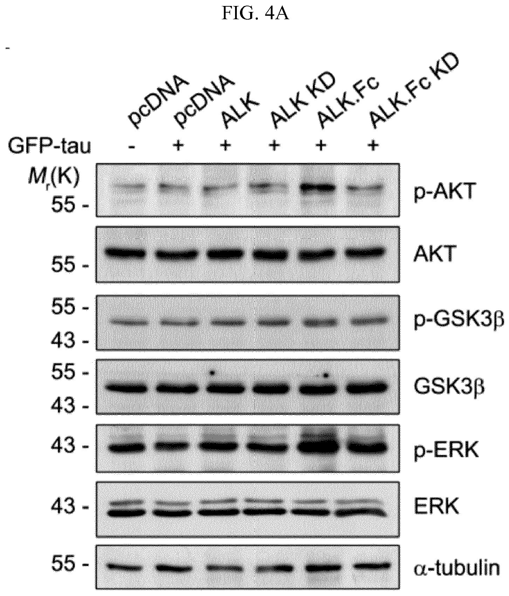

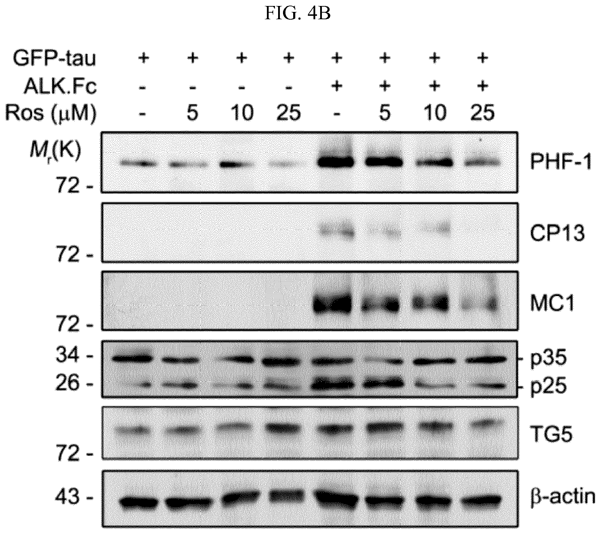

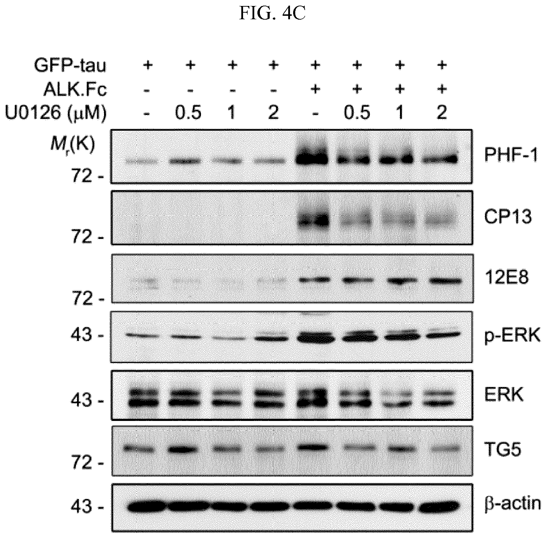

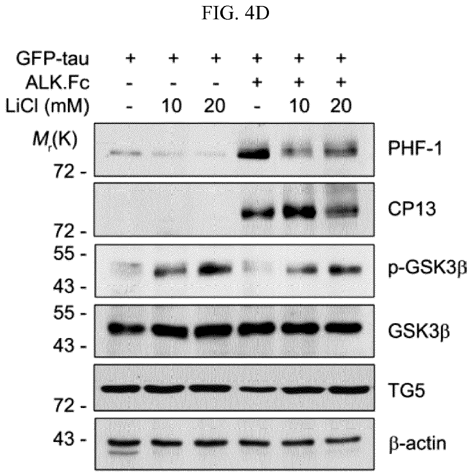

FIG. 4A shows a western blot result examining that AKT and ERT are activated by ALK; FIGS 4B to 4D show western blot results examining that ALK-mediated tau phosphorylation is respectively inhibited by roscovitine (Ros) (FIG. 4B), U0126 (FIG. 4C), and LiCl (FIG. 4D) which are kinase inhibitors;

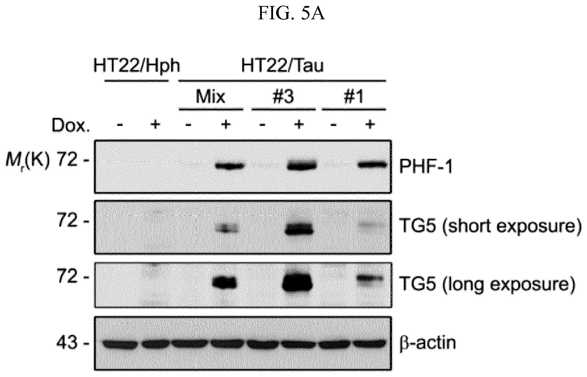

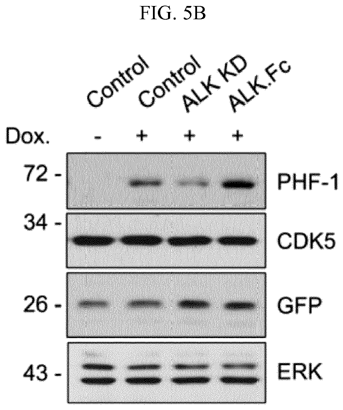

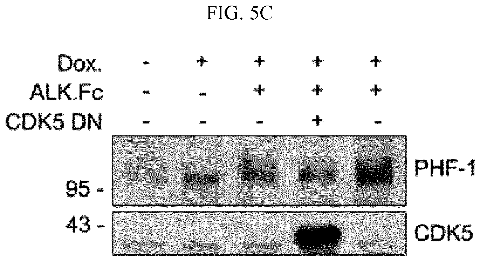

FIG. 5A shows a western blot result examining that tau expression is induced by doxycycline treatment in a tau-transformed HT22 cell line which stably expresses tau; FIG. 5B shows a western blot result examining that tau phosphorylation is increased when HT22/Tau #1 cell line is transfected with pALK.Fc construct; FIG. 5C shows a western blot result examining that ALK-mediated tau phosphorylation is inhibited by a CDK5 dominant-negative variant;

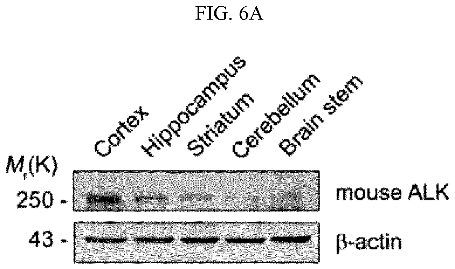

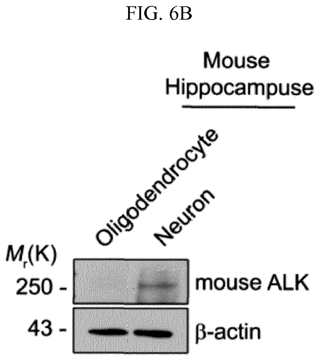

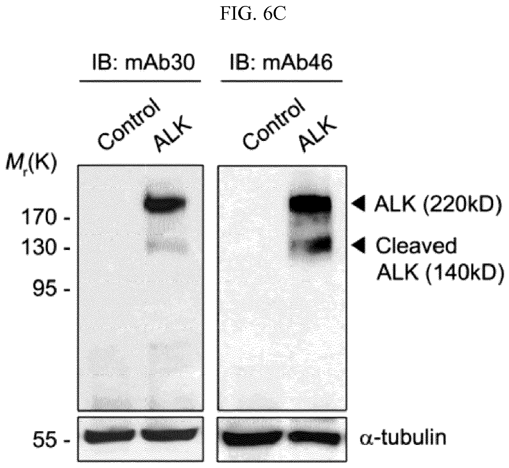

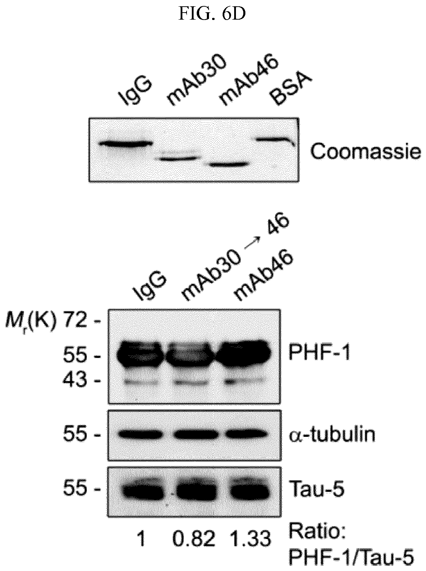

FIG. 6A shows a western blot result examining an ALK expression level in brain tissue of PI mice; FIG. 6B shows a western blot result examining ALK expression in primary neuronal cells of P1 mice; FIG. 6C shows a western blot result examining specificity of ALK to mAb46 and mAb30 which have agonistic or antagonistic action to ALK; FIG. 6D shows a western blot result examining that phosphorylation of tau is increased by mAb46 and inhibited by mAb30 in mouse primary neuronal cells;

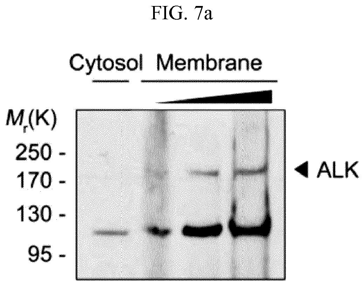

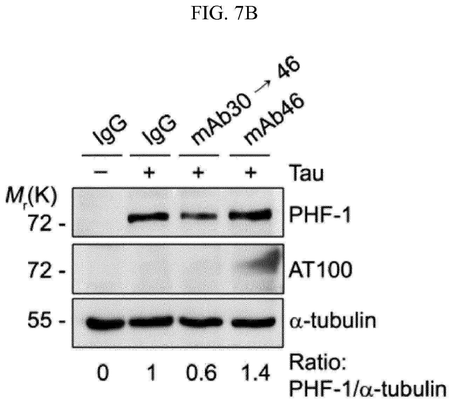

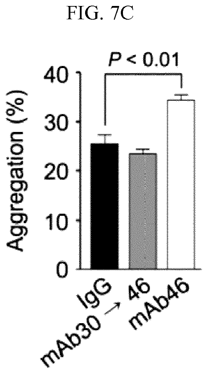

FIG. 7A is a western blot image examining ALK expression in a membrane fraction of HT22 cells; FIG. 7B shows a western blot result examining that tau phosphorylation is increased by mAb46 and inhibited by mAb30 in HT22 cells; FIG. 7C is a graph showing a result of examining a tau aggregation level through a fluorescence microscopy;

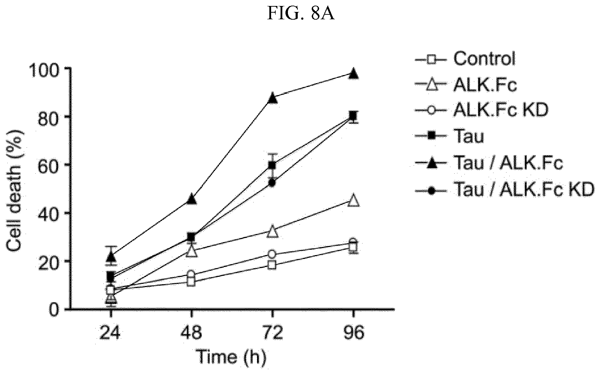

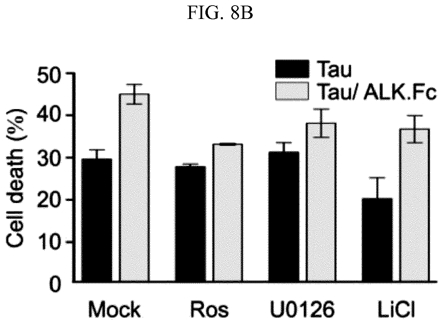

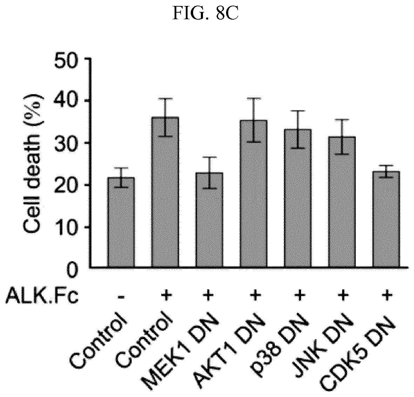

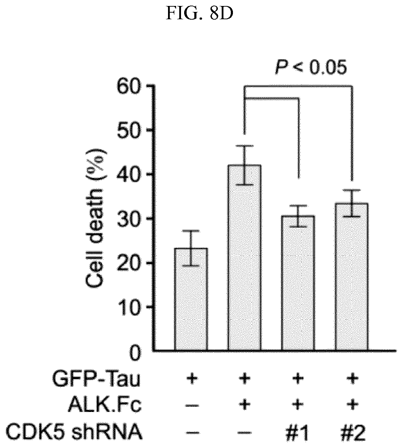

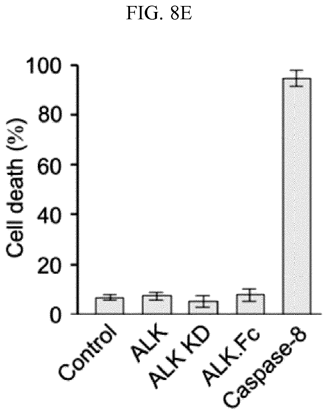

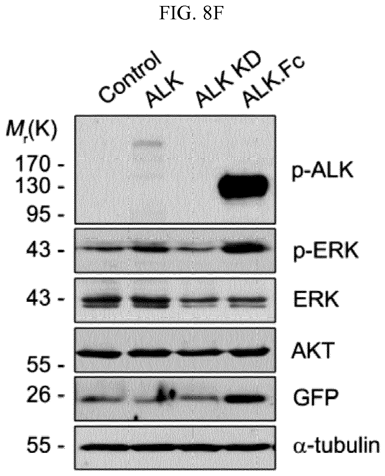

FIG. 8A is a graph showing that tau-mediated neuronal cell death is increased by ALK; FIG. 8B is a graph showing that ALK-mediated cytotoxicity of tau is dependent on ERK and CDK5; FIG. 8C is a graph showing that ALK/tau-mediated cell death is inhibited by a CDK4 or MEK1 dominant-negative variant; FIG. 8D is a graph showing that ALK/tau cytotoxicity is inhibited by CDK5 shRNA; FIG. 8E is a graph showing that ALK/tau toxicity is not exhibited in non-neuronal cells; FIG. 8F shows a western blot result of cells observed in FIG. 8E;

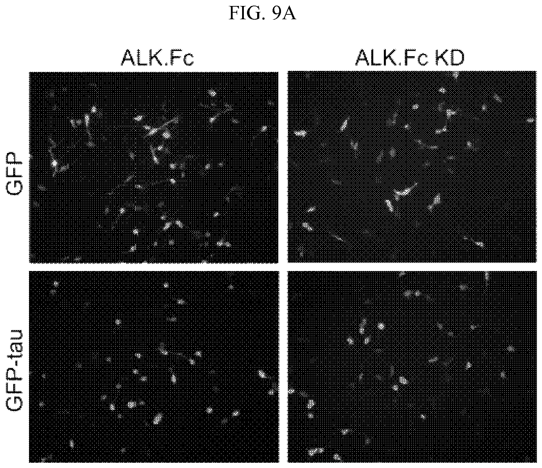

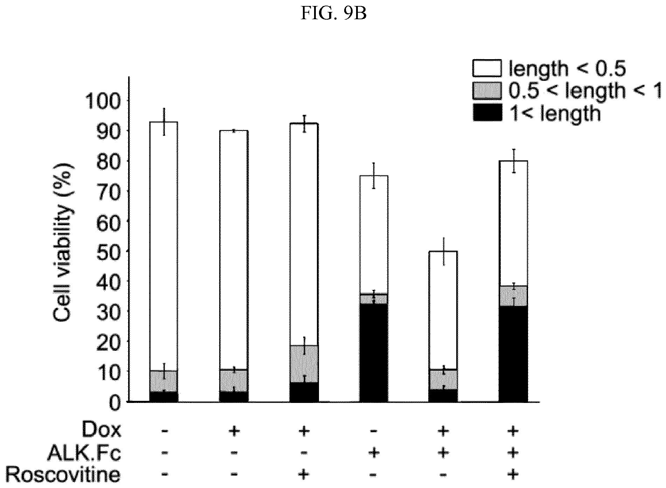

FIG. 9A are images observed by a fluorescence microscopy showing a damaged neurite due to ALK and tau expression in HT22 cells; FIG. 9B is a graph showing that the CKD5 inhibitor, roscovitine, recovers cell death and damaged neurite growth in tau-transformed HT22 cell line;

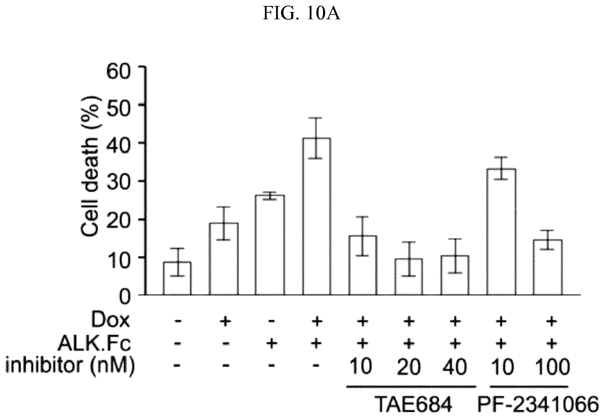

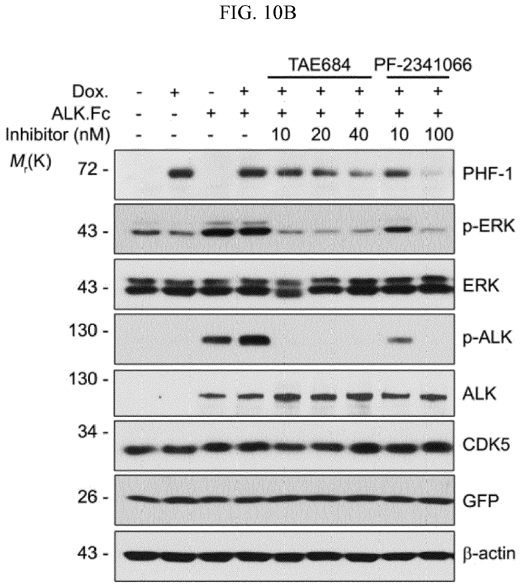

FIG. 10A is a graph showing the effect of an ALK-specific inhibitor on ALK-mediated tau neurotoxicity inhibition; FIG. 10B is a western blot result of cells treated with the ALK-specific inhibitor;

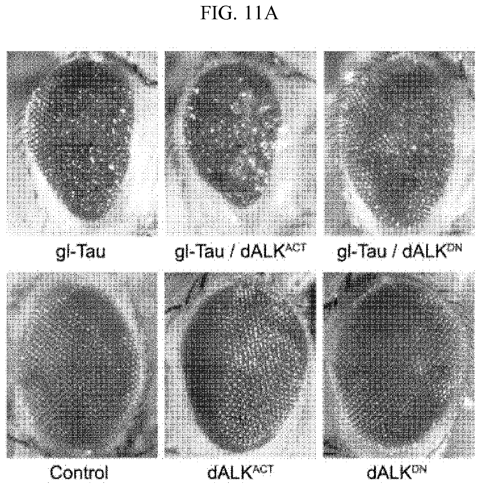

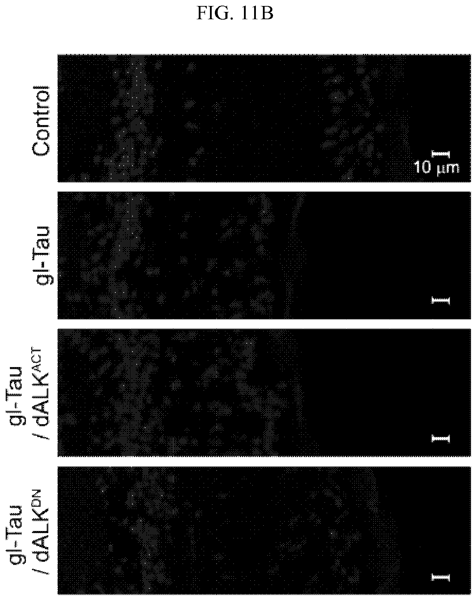

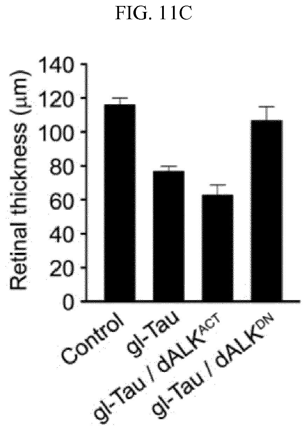

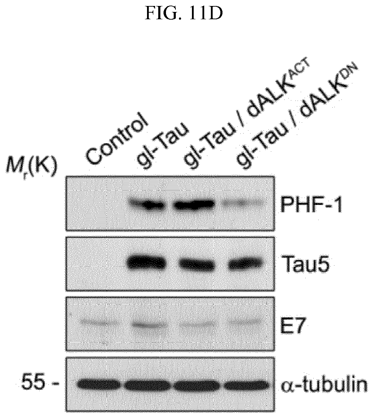

FIG. 11A are microscopic images showing that rough-eye phenotype and an irregular shape of an ommatidium in tau-transformed fruitfly are increased when structurally activated fruitfly ALK is coexpressed; FIG. 11B are microscopic images showing deterioration in inner neuronal degeneration of a fruitfly; FIG.11C is a graph obtained by digitizing a neuronal degeneration level observed in FIG. 11B; FIG. 11D shows a western blot result examining that a tau phosphorylation level of a tau-transformed fruitfly is regulated by dALK^{ACT} and dALK^{DN};

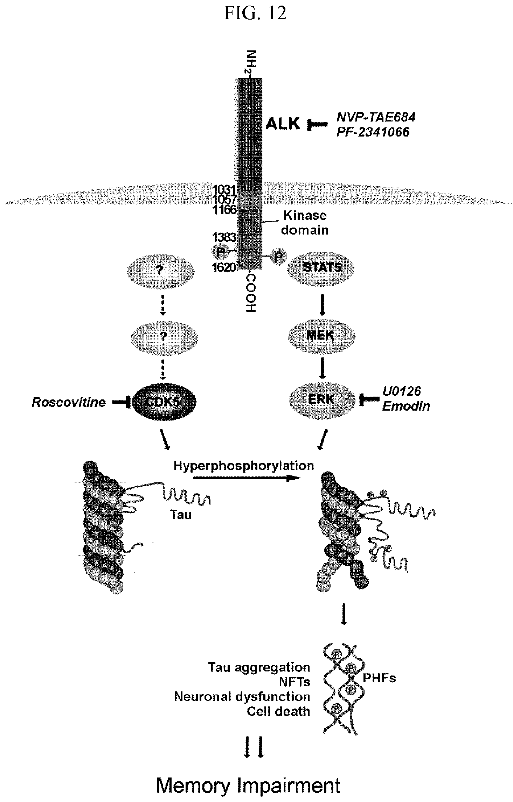

FIG. 12 is a schematic diagram illustrating that ALK induces tau phosphorylation and neuronal cell death through CKD5 and ERK activation;

### DETAILED DESCRIPTION OF EMBODIMENTS

The terms used herein are defined as follows.

As used herein, "tau" is a microtubule-associated protein. It has been known that abnormal hyperphosphorylation and aggregation of tau cause a degenerative neuronal disease (see Lee et al., Annu. Rev. Neurosci., 24: 1121-1159, 2001; Bergeron et al., J. Neuropathol. Exp. Neurol., 56: 726-734, 1997; Bugiani et al., J. Neuropathol. Exp. Neurol., 58: 667-677, 1999; Delacourte et al., Ann. Neurol., 43: 193-204, 1998; Ittner and Gotz, Nat. Rev. Neurosci., 12: 65-72, 2011).

As used herein, "PHF-1", "12E8", "AT8", and "AT100" are antibodies which recognize phosphorylation of tau protein, wherein PHF-1 recognizes phosphorylation at the position of pSer396/404; 12E8 recognizes phosphorylation at the position of pSer262/356; AT8 recognizes phosphorylation at the position of pSer202/pThr205; and AT100 recognizes phosphorylation at the position of pThr212/pSer214. A position in tau protein where phosphorylation occurs relates to type of neurofibrillary tangle (NFT) and a symptom of a degenerative neural disease.

As used herein, "ALK" means anaplastic lymphoma kinase. It has been known that abnormal ALK activity causes a cancer in a fusion protein form in an inflammatory myofibroblastic cancer, diffuse large B-cell lymphoma, and anaplastic large-cell lymphoma, and ALK protein alternation leads occurrence of familiar neuroblastoma (see Iwahara et al., Oncogene, 14:439-449, 1997; Morris et al., Oncogene, 14: 2175-2188, 1997; Chen et al., Nature 455: 971-974, 2008; George et al., PLoS One 2: e255, 2007; Morris et al., Science, 263: 1281-1284, 1994.). It has been known that Ras/MEK/ERK, PI3K/AKT, and JAK3/STAT3 serve as downstream signaling molecules thereof.

As used herein, "CDK5" means cyclin-dependent kinase5 (CDK5) which has been known to play important roles in synapse plausibility, neurite growth, and neural development (see Nikolic et al., Genes Dev. 10(7): 816-825, 1996; Paglini et al., J. Neurosci. 189(23): 9858-9869, 1998). It has been known that abnormal activation of CDK5 leads hyperphosphorylation of tau thereby reducing the ability of tau to bind microtubules which resultantly causes NFT formation (Wen et al., Biochim. Biophys. Acta., 1772(4): 473-83, 2007).

As used herein, "a transformed plant" or "a transformed animal" means a genetically engineered plant or animal to express a heterologous gene by introducing the heterologous gene into a genome or to have deletion in a certain gene such that the gene is not expressed. Typically, a transformed animal may be produced by genetically engineering a germinal cell and also through an animal cloning pharmaceutical composition by genetically engineering a somatic cell followed by a nuclear substitution. A transformed plant may be more simply produced by infecting somatic cells with agrobacteria including a heterologous gene followed by dedifferentiation and re-differentiation processes. The pharmaceutical compositions for producing a transformed animal and a transformed plant are well known in the art (Jaenisch, R and B. Mintz, Proc. Natl. Acad. Sci. USA, 71(4): 1250-1254, 1974; Cho et al., Curr. Protoc. Cell Biol., 42: 19.11.1-19.11.22, 2009; Johnston, S. A. and D. C. Tang, Meth. Cell Biol., 43 Pt A: 353-365, 1994; Sasaki et al., Nature 459(7246): 523-527, 2009; Vaek et al., Nature, 328(6125): 33-37, 1987).

Hereinafter, examples of the present invention will be described with reference to the accompanying drawings. However, the present invention is not limited to following examples shown in accompanying drawings, and can be implemented in variously different forms. The examples showed in figures hereinafter complete the disclosure of the present invention, and are provided to completely notify a scope of the present invention to a person skilled in the art. Also, for the convenience of the description, the size of an element can be exaggerated or reduced in figures.

FIG. 1A is a graph showing a result of counting aggregated HT22 cells through a fluorescence microscopy when HT22 cells were cotransfected with pGFP-tau and one of pcDNA, pGSK3*β* CA, and pmALK. FIG. 1B shows a western blot result examining that tau phosphorylation is increased as an amount of treated pmALK increases in cells observed in FIG. 1A. FIG. 1C is a graph showing that the number of aggregated HT22 cells, which is reduced when pGFP-tau and pmALK are cotransfected and then emodin treatment is followed, is counted through a fluorescence microscopy. FIG. 1D shows a western blot result to examine a change in a phosphorylation level of tau depending on an increase in an amount of treated emodin in cells observed in FIG. 1C. By using a cell-based functional screening pharmaceutical composition using pGFP-tau of which availability is validated through an example of the present invention the effect of selected ALK of a mouse is examined using phosphorylation of tau as a regulation factor. Aggregation of tau is significantly increased when pmALK construct is cotransfected into HTT cells with pGFP-tau when compared to where pmALK construct is cotransfected with GSK3*β* CA (FIG. 1A). It has been reexamined through a western blot result showing an increase in PHF-1 (FIG. 1B), wherein the western blot is carried out after extracting the observed cells and then using the extracted cells. In addition, aggregation and phosphorylation levels of tau are reduced when emodin treatment is performed after pGFP-tau and pmALK constructs are cotransfected, wherein, emodin is an inhibitor of PHF aggregation and ERK activation (FIG. 1C). A phosphorylation level of tau is examined using PHF-1 antibody and tau-1 antibody, wherein PHF-1 antibody recognizes a phosphorylated tau and tau-1 antibody recognizes an unphosphorylated state of tau. It has been examined that PFH-1 expression is decreased and tau-1 expression is increased as an amount of treated emodin increases (FIG. 1D).

FIG. 2A shows a western blot result examining that phosphorylation of HT22 cells is increased by cotransfection of pGFP-tau and pALK. FIG. 2B is a graph showing that the number of aggregated HT22 cells, which is increased as an introduced amount of pALK increases when HT22 cells are cotransfected with pGFP-tau and pALK, is counted by a fluorescent microscopy. FIG. 2C are fluorescence microscopy images showing that phosphorylation and aggregation of HT22 cells are increased by cotransfection of pHA-Tau and pALK. To determine whether human ALK gene may facilitate phosphorylation and aggregation of tau as mouse ALK does, HT22 cells are cotransfected with pALK and pGFP-tau constructs. Through western blot, it was examined that expression of PHF-1, 12E8 is increased. This means Ser396/404 (PHF-1), and Ser262/356 (12E8) of tau are phosphorylated. Moreover, observing the result through a fluorescence microscopy, aggregation of tau is increased, and aggregation is become higher as an amount of pALK increases. The human ALK make tau be more aggregated than GSK3β does, and the result is examined again through an immunostaining result of cells cotransfected with pALK and pGFP-tau. The result demonstrates that human ALK may facilitate phosphorylation and aggregation of tau.

FIG. 3A is a schematic diagram illustrating wild-type human ALK and ALK variant constructs. FIG. 3B is a graph showing that aggregated HT22 cells are reduced when HT22 cells are cotransfected with pGFP-tau and one of ALK constructs (i.e., pALK, pALK KD, pALK.Fc, and pALK.Fc KD) thereby inactivating ALK through a fluorescence microscopy. FIG. 3C is a western blot result of cells observed in FIG. 3B. When HT22 cells are cotransfected with pALK KD and pALK.Fc KD constructs in which kinase activity of human ALK is inactivated, a decrease in cell aggregation is observed through a fluorescence microscopy and decreases in expression of proteins indicative of tau phosphorylation such as PHF-1, CP13, 12E8 and also in phosphorylation of ALK are observed through western blot. The result demonstrates that kinase activity of human ALK plays important roles in phosphorylation and aggregation of tau.

FIG. 4A shows a western blot result examining that AKT and ERK are activated by ALK. FIGS 4B to 4D show western blot results examining that ALK-mediated tau phosphorylation is inhibited by roscovitine (Ros) (FIG. 4B), U0126 (FIG. 4C), and LiCl (FIG. 4D) each of which are kinase inhibitors. After HT22 cells are cotransfected with pGFP-tau and pALK.Fc, phosphorylation levels of AKT and ERK proteins are increased, wherein, the AKT and ERK proteins are known as downstream kinase of ALK in cancer cells. Also, phosphorylation of tau is significantly reduced in a manner dependent on an amount of treated roscovitine and U0126, wherein roscovitine is a CDK5 inhibitor and U0126 is an ERK inhibitor. These results demonstrate that ALK increases phosphorylation through ERK and CDK activation.

FIG. 5A shows a western blot result examining that tau expression is induced by doxycycline treatment in a tau-transformed HT22 cell line which stably expresses tau. FIG. 5B shows a western blot result examining that tau phosphorylation is increased when a HT22/Tau #1 cell line is transfected with pALK.Fc construct. FIG. 5C shows a western blot result examining that ALK-mediated tau phosphorylation is inhibited by a CDK5 dominant-negative variant. As a result of reexamining that phosphorylation of tau is increased by ALK by using a HT22 neuronal cell line which stably expresses tau, it has been examined that HT22/tau transformed cells normally express tau protein, and then, phosphorylation of tau is increased when the HT22/Tau #1 cell line is transfected with the pALK.Fc construct and thereafter treated with doxycycline. In addition, it has been examined that phosphorylation of tau is increased by ALK-mediated CDK5 when the HT22/Tau #1 cell line is cotransfected with pALK.Fc and pCDK5 DN (D144N). These results correspond to the result observed in Experimental Examples 2, and 3.

FIG. 6A shows a western blot result examining an ALK expression level in brain tissue of PI mice. FIG. 6B shows a western blot result examining ALK expression in primary neuronal cells of PI mice. FIG. 6C shows a western blot result examining specificity of ALK to mAb46 and mAb30 which have agonistic or antagonistic action to ALK. FIG. 6D shows a western blot result examining that phosphorylation of tau is increased by mAb46 and inhibited by mAb30 in mouse primary neuronal cells. To examine the effect of endogenous ALK on tau phosphorylation, ALK expression is examined in brain tissue of a mouse and primary cells, which are isolated therefrom and cultured, at postnatal day 1 (PI). Then, primary cells treated with ALK-specific mAb46 and mAb30 antibodies. Consequently, it has been observed that phosphorylation of tau is increased by mAb46 treatment, and phosphorylation of tau is offset in a group treated with mAb30 followed by mAb46. These results demonstrate that endogenous ALK is involved in an increase in tau phosphorylation.

FIG. 7A shows a western blot image examining ALK expression in a membrane fraction of HT22 cells. FIG. 7B shows a western blot result examining that tau phosphorylation is increased by mAb46 and inhibited by mAb30 in HT22 cells. FIG. 7C is a graph showing the result of examining an aggregation level of tau through a fluorescence microscopy. After ALK expression is examined in a membrane fraction of HT22 cells, cells are treated with either mAb46 or mAb30. Then, a phosphorylation level of tau is examined through western blot and aggregation level of tau is examined through a fluorescence microscopy. Consequently, phosphorylation and aggregation of tau are increased by mAb46 having an agonistic action which is the same as the observed result of PI mouse primary cells.

FIG. 8A is a graph showing that tau-mediated neuronal cell death is increased by ALK. FIG. 8B is a graph showing that ALK-mediated cytotoxicity of tau is dependent on ERK and CDK5. FIG. 8C is a graph showing that ALK/tau-mediated cell death is inhibited by CDK4 or a MEK1 dominant-negative variant. FIG. 8D is a graph showing that ALK/tau cytotoxicity is inhibited by CDK5 shRNA. FIG. 8E is a graph showing that ALK/tau toxicity is not exhibited in non-neuronal cells. FIG. 8F shows a western blot result of cells observed in FIG. 8E. It has been examined that neuronal cell death is increased when HT22 cells are cotransfected with pALK.Fc and pGFP-tau, and the cell death is dependent on ERK, and CDK5 by using a kinase inhibitor, a dominant-negative variant construct, and CDK5 shRNA. Also, it has been examined that ALK/Tau cell death is induced in a neuronal cell specific manner, because cell death is not induced in human breast cancer cell line, MCF7 which is cotransfected with pALK.Fc and pGFP-tau. These results correspond to the observed result of Experimental Example, and further demonstrate that ALK-mediated tau phosphorylation and cell death arises in a neuronal cell-specific manner.

FIG. 9A are images observed by a fluorescence microscopy showing damaged neurite caused by ALK and tau expression in HT22 cells. FIG. 9B is a graph showing that a CKD5 inhibitor, roscovitine, recovers cell death and damaged neurite growth in a tau-transformed HT22 cell line. It has been known that ALK recovers neurite growth in neuronal cells and a fruitfly model (Motegi et al., J. Cell. Sci., 117, 3319-3329, 2004), however, when expressed with tau, neurite growth facilitation is not observed. When ALK.Fc is expressed in the tau-transformed HT22 cell line which is treated with a CDK5 inhibitor, roscovitine, the effect of ALK/Tau on neurite growth is recovered. These results demonstrate that CKD5 is involved in ALK-mediated neurite growth and neurotoxicity.

FIG. 10A is a graph showing the effect of an ALK-specific inhibitor on ALK-mediated tau neurotoxicity inhibition. FIG. 10B shows a western blot result of cells treated with an ALK-specific inhibitor. HT22/Tau #1 cells are transfected with pALK.Fc, and then tau expression is induced by doxycycline. Thereafter cells are treated with an ALK-specific inhibitor and observed with a fluorescence microscopy. Then, cells are extracted to perform western blot. As a result, cell death induced by ALK/Tau is reduced due to the ALK inhibitor treatment, and cell death is inhibited in a manner dependent on an amount of treated ALK. These results demonstrate that kinase activity of ALK is needed for both tau phosphorylation and tau-mediated neurotoxicity.

FIG. 11A are microscopic images showing that rough-eye phenotype and an irregular shape of an ommatidium in a tau-transformed fruitfly are increased when structurally activated fruitfly ALK is co-expressed. FIG. 11B are microscopic images showing deterioration in inner neuronal degeneration of a fruitfly. FIG.11C is a graph obtained by digitizing a neuronal degeneration level observed in FIG. 11B. FIG. 11D is a western blot result showing that a tau phosphorylation level of a tau-transformed fruitfly is regulated by dALK^{ACT} and dALK^{DN}. The present inventor investigates the previously examined *in vitro* effects of ALK, which is tauphosphorylation and aggregation mediation, and neuronal cell-specific cell death mediation, by using an *in vivo* fruitfly model. As a result, a tau-transformed fruitfly produced by using dALK^{ACT}, which retains kinase activity of ALK, shows a phenotype in which the size of eyes of fruitfly is reduced and a shape of eyes is irregular. In addition, neuronal degeneration is observed in a retina of eyes and also phosphorylation of tau is increased simultaneously. These results demonstrate that the function of ALK activity as kinase, examined *in vitro,* also plays important roles in phosphorylation and aggregation of tau and neurotoxicity *in vivo.*

FIG. 12 is a schematic diagram illustrating that ALK induces tau phosphorylation and neuronal cell death through CKD5 and ERK activation. As summary of the result demonstrated by an example of the present invention, it shows that activated ALK activates CKD5 and ERK, and increases phosphorylation and aggregation of tau, thereby inducing neuronal cell death.

Hereinafter, the present invention will be described in more detail with reference to Examples and Experimental Example. However, the present invention is not limited to Examples and Experimental Examples disclosed hereinafter, and can be achieved in various aspects different from each other. The Examples and Experimental Examples hereinafter complete the disclosure of the present invention, and are provided to completely notify a scope of the present invention to a person skilled in the art.

### EXAMPLE 1: CELL CULTURE PHARMACEUTICAL COMPOSITION

### 1-1: HT22 CELL AND HeLa CELL

Mouse hippocampus HT22 and HeLa cells were cultured in Dulbecco's Modified Eagles Medium (DMEM) (Invitrogen) including 10% (v/v) fetal bovine serum (FBS) (Invitrogen).

### 1-2: TAU-TRANSFORMED HT22 CELL LINE (STABLE CELL LINE)

HT22 cells were transfected with pBIG2i-tau construct. One day after, the cells were cultured in a medium including 200 g/ml of hygromycin B (Clontech) for two weeks to produce a HT22/Tau mixed cell group. Monoclonal HT22/Tau #1 and #3 were separated from the HT22/Tau mixed cell group, and treated with 1 µg/ml of doxycycline (Dox) (Sigma, USA) to express tau.

### 1-3: PRIMARY NEURONAL CELL CULTURE

A brain of a mouse was isolated on postnatal day 1 (PI) to culture primary mouse hippocampus neuronal cells. Briefly, hippocampus tissue of the isolated mouse brain was treated with trypsin, and then primary neuronal cells were obtained from neuronal cells through a concentration gradient pharmaceutical composition according to the Optiprep pharmaceutical composition (Brewer and Torricelli, Nat Protoc 2, 1490-1498, 2007). Cells were then transferred to a neuronal basal medium including B27 serum supplement (Invitrogen) and cultured on a 12-well tissue culture plate in a condition of 2 x 10⁶ cells per well.

### EXAMPLE 2: PRODUCTION OF TRANSFORMED FRUITFLY

### 2-1: gl-TAU2.1 FRUITFLY LINE

A fruitfly (*Drosophila melanogaster*) of gl-Tau2.1 line was provided from Dr. Daniel Geschwind (University of California-Los Angeles, CA), wherein the fruitfly expresses wild-type human tau4R gene within a pExpress-gl-modified GMR expression vector and exhibits normal phenotype of 3rd chromosome.

### 2-2: UAS-Tau FRUITFLY LINE (UAS-dALK^{FL}, UAS-dALK^{ACT} and UAS-dALK^{DN})

UAS-Tau fruitfly, which was used to produce elav-tau, was provided from Dr. Mel Feany (Wittmann et al., Science, 293, 711-714, 2001). A fruitfly (UAS-dALK^{FL}), which is a wild-type, full-length fruitfly anaplastic lymphoma kinase (ALK) line, and fruitfly ALK modified lines (UAS-dALK^{ACT} and UAS-dALK^{DN}) were respectively provided form Dr. Ruth Palmer (the Salk Institute, CA) and Manfred Frasch (Mount Sinai School of Medicine, NY) (see Lee et al., Nature, 425, 507-512, 2003; Loren et al., EMBO Rep, 4, 781-786, 2003; Loren et al., Genes Cells, 6, 531-544, 2001). Briefly, structurally active ALK (UAS-dALK^{ACT}) codes for a construct into which 1-117 codon of human nucleophosmin (NPM) (Genebank Accession number: P06748) and 1129-1801 codon of fruitfly ALK are fused. The UAS-dALK^{DN}, which is a dominant-negative ALK construct, encodes an extracellular domain, a transmembrane domain, and a short tail portion of an intracellular domain of fruitfly ALK. Fruitflies were cultured in a standard corn dietary-based fruitfly medium at 25°C°C. Adult fruitfly was used for analysis for five days after ecdysis.

### EXAMPLE 3: VECTOR CONSTRUCTION

### 3-1: PRODUCTION OF pHA-tau, pGFP-tau, pGFP-tau_{D421} AND pBIG2i-tau CONSTRUCTS

The mammalian expression pCI vector, which expresses the longest-type human tau (2N4R) (Genebank Accession number: NM_005910) was provided from Dr. Akihiko Takashima (RIKEN, Japan).

Tau cDNA was obtained by using the provided vector. Then, tau cDNA was subcloned into pcDNA3-HA (Invitrogen), pGFP (Clontech), and pBIG2i vectors which include a tetracycline-regulated promoter for regulating expression of an inserted gene, and a selection marker such as GFP fusion protein or hygromycin B which produces HA (Krishnamurthy et al., J. Biol. Chem., 279, 7893-7900, 2004). Consequently, pHA-tau, pGFP-tau, and pBIG2i-tau constructs were produced.

Further, pGFP-tau_{D421} was produced by cloning tau to have a caspase portion-truncated form and exclude a polynucleotide coding amino acids corresponding to amino acids 422-441 of human tau (2N4R) (GenBank Accession number: NM_005910).

### 3-2: PRODUCTION OF MOUSE pmALK CONSTRUCT

The mammalian pME18S-FL3 vector expressing mouse ALK (GenBank Accession number: D83002) was provided from Dr. Tadashi Yamamoto of Tokyo University (Iwahara et al., Oncogene 14: 439-449, 1997).

### 3-3: PRODUCTION OF HUMAN pALK^{FL}, AND pALK KD CONSTRUCTS

Human pALK^{FL} and pALK KD constructs were provided from Dr. Anton Wellstein (Georgetown University, Washington) (Kuo et al., Oncogene 26: 859-869, 2007). The human pALK^{FL} construct was produced by subcloning full-length human ALK (GenBank Accession number: U62540) into pcDNA3.1/Myc-His vector, and the human pALK KD construct was a kinase inactivated variant (ALK kinase-dead, hereinafter, abbreviated as ALK KD) produced by subcloning into pcDNA3.1/Myc-His vector to have an alternation in the unchangeable lysine residue (K1150) positioned at the ATP binding site into alanine.

### 3-4: PRODUCTION OF HUAMN pALK.Fc, AND pALK.Fc KD CONSTRUCTS

Human pALK.Fc and pALK.Fc KD constructs were provided from Dr. Mel Vigny (INSERM U440, Paris) (Souttou et al., J. Biol. Chem., 276: 9526-9531, 2001). The human pALK.Fc construct includes mouse IgG 2b Fc instead of an extracellular domain of a receptor in pcDNA3.1 plasmid, and the human ALK.Fc KD construct has a form in which ALK kinase of the pALK.Fc construct is inactivated.

The pALK.Fc and pALK.Fc KD were subcloned into pCSII-EF-MCS-IRES2-Venus lentivirus vector and used to produce lentivirus. The resultants were respectively referred to as pLenti-ALK.Fc and pLenti-ALK.Fc KD.

### 3-5: PRODUCTION OF GSK3β CA (S9A), MEK1 DN (K97R), AKT1 DN (K179M), AND CDK5 DN (D144N) MUTANTS

GSK3β CA (S9A), MEK1 DN (K97R), AKT1 DN (K179M), and CDK5 DN (D144N) were produced by using synthesized oligonucleotides for producing each variant with a Quickchange Site-Directed Mutagenesis kit (Stratagene), wherein: GSK3β CA(S9A) was produced by substituting amino acid serine at the position 9 of GSK3β (GenBank Accession number: NM_002093) with alanine; MEK1 DN (K97R) was produced by substituting amino acid lysine at the position 97 of MEK1 (GenBank Accession number: NM_002755) with arginine; AKT1 DN (K179M) was produced by substituting amino acid lysine at the position 179 of AKT1 (GenBank Accession number: NM_001014432) with methionine; and CDK5 DN (D144N) was produced by substituting amino acid aspartic acid at the position 144 of CDK5 (GenBank Accession number: NM_004935) with asparagines. All variants were examined through DNA sequencing.

### EXAMPLE 4: PRODUCTION OF LENTIVIRUS

A lentivirus stock was produced by introducing pMDLg/pRRE and pCMV-VSV-G-RSV-Rev, which are modified transfer vector and packing vector, into HEK 293FT cells using calcium phosphate. After 48 to 60 hours of infection, supernatant of HEK 293FT cells was obtained and then concentrated through ultracentrifugation for 2 hours under the condition of 4°C and 50,000 x g. The concentrated virus stock was serially diluted in HEK293FT cells and virus titer was measured after cells were infected.

### EXPERIMENTAL EXAMPLE 1: EXAMINATION OF INCREASE IN TAU AGGREGATION AND PHOSPHORYLATION BY MOUSE ALK

To screen a novel gene which regulates aggregation and phosphorylation of tau, 630 complementary DNA clones of kinase were coexpressed with tau_{D421} by using a gain of function screening pharmaceutical composition. Consequently, mouse anaplastic lymphoma kinase (ALK) was identified as a novel gene regulating aggregation and phosphorylation of tau.

### 1-1: EXAMINATION OF INCREASE IN TAU AGGREGATION AND PHOSPHORYLATION ACCORDING TO INCREASE IN AMOUNT OF TREATED MOUSE ALK

To examine whether mouse ALK increases aggregation and phosphorylation of tau, a pGFP-tau-based functional screening pharmaceutical composition of which availability was validated in the Experiment Example 1 was used. Specifically, HT22 cells were cotransfected with pGFP-tau and one of pcDNA (a negative control), pGSK3β CA (a positive control), or pmALK. The transfection was performed while amounts of cotransfected pcDNA, pGSK3β CA, and pmALK constructs were increased to 100, 200, and 400 ng. After 24 hours of transfection, aggregation of tau was examined as the number of GFP-positive cells appeared to be aggregated by using a fluorescence microscopy. After then, the cells were extracted to examine phosphorylation of tau by analyzing PHF-1 protein expression with western blot.

Observing the cotransfected cells through a fluorescence microscopy, as shown in FIG. 2A, a group cotransfected with pGSK3β CA or pmALK exhibits a significant increase in aggregation of tau than a group cotransfected with pcDNA and pGFP-tau construct. Also, tau aggregation was significant when pmALK construct than pGSK3β CA was cotransfected, wherein pGSK3β CA constantly shows structural activity (FIG. 2A). Statistical analysis was performed using the SigmaPlot program. The result was shown as mean ± S.D of triplicate experiments. P-value was calculated against the control by using the t-test pharmaceutical composition (#P<0.001; *P<0.01;**P<0.05).

In addition, as a result of performing western blot after extracting cells observed in FIG. 1A with a fluorescence microscopy, PFH-1 expression, which is an indicative of tau phosphorylation, increased as an amount of cotransfected mALK to 0, 0.1, 0.2, and 0.4 µg, increased.

### 1-2: EXAMINATION OF INCREASE IN ALK-MEDIATED TAU PHOSPHORYLATION AND AGGREGATION BY EMODIN

Subsequently, the present inventor examined aggregation and phosphorylation of tau caused by coexpression of mouse tau and mouse ALK as observed in Experimental Example 1-1 through treatment of an inhibitor of ERK activation and PHF aggregation. Specifically, HT22 cells were cotransfected with pmALK and pGFP-tau constructs, and condensation of cells depending whether emodin was treated or not was counted, wherein emodin is an inhibitor of ERK activation and PHF aggregation. Then, the cells were extracted to examine a phosphorylation level of tau protein by using PHF-1 antibody and Tau-1 antibody.

As a result of observing the cotransfected cells with a fluorescence microscopy, as shown in FIG. 2C, tau aggregation caused by ALK was inhibited by emodin treatment (IC₅₀ value, 1-5 µM) (see FIG. 2C, Mijatovic et al., Cell Mol. Life Sci., 62: 1275-1282, 2005; Pickhardt et al., J. Biol. Chem., 280: 3628-3635, 2005).

Moreover, emodin treatment increased tau-1 expression while decreasing PHF-1 expression, and a total amount of transfected tau, which was examined by using anti-tau 12 antibody, was relatively constant. Accordingly these results demonstrate that mouse ALK increases phosphorylation at the position of Ser³⁹⁶/Ser⁴⁰⁴ (PHF-1).

### EXPERIMENTAL EXAMPLE 2: EXAMINATION OF INCREASE IN TAU PHOSPHORYLATION AND AGGREGATION BY HUMAN ALK

To examine whether human ALK serves the same role as mouse ALK in tau modification, the present inventor observed influence of human ALK on phosphorylation and aggregation of tau in neuronal cells.

### 2-1: EXAMINATION OF INCREASE IN TAU AGGREGATION AND PHOSPHORYLATION BY HUMAN ALK

HT22 cells were cotransfected with pGFP-tau and pALK. To examine influence of pALK on phosphorylation and aggregation of tau, transfection was performed by increasing an amount of transfected pALK. After 24 hours, a cell extract was obtained. Then, an abnormal phosphorylation level of tau and ALK activation level were examined with western blot. The used antibodies are as follow: PHF-1 (p-Ser396/404; Davies), 12E8 (p-Ser262/356; provided from Dr. Peter Seubert (Elan Pharmaceuticals)), Tau-5 (total Tau; Biosource, AHB0042), p-ALK (p-Tyr1604; Cell signaling, #3341), p-ERK (Cell signaling, #9101), ERK (Cell signaling, #9102), and p-GSK3*β* (p-Ser9; Cell signaling, #9323).

Consequently, it has been observed that expression of PHF-1 and 12E8 was increased and phosphorylation of ALK, ERK and GSK3β was increased. The result demonstrates that various epitope of tau was phosphorylated by human ALK and human ALK increases phosphorylation of tau as mouse ALK does (FIG. 2A).

Subsequently, influence of human ALK on phosphorylation and aggregation of tau was compared to that of GSK3β. HT22 was cotransfected with pGFP-tau and pALK or pGSK3*β* CA, and then aggregation of tau was measured by counting the number of GFP-positive cells appeared to be aggregated by using a fluorescence microscopy. Consequently, tau aggregation was increased in cells cotransfected with pGFP-tau and pALK than cells cotransfected with GSK3*β* CA, and also, the effect was significant as an amount of introduced pALK increases (FIG. 2B). The value shown in FIG. 2B was mean ±S.D (n=4), and P-value was calculated by using the t-test pharmaceutical composition.

Further, it was reexamined that human ALK facilitates phosphorylation and aggregation of tau protein through immunostaining pharmaceutical composition. HT22 cells were transfected with pcDNA construct alone, pHA-Tau construct alone, or pHA-Tau and pALK constructs, and then cells were stained with PHF-1 antibody and thioflavin-S antibody to examine phosphorylation and aggregation of tau protein. Fluorescence microscopic images of the cells were obtained with a fluorescence microscopy, and then overlay images were prepared by overlapping staining images of respective antibodies. Consequently, thioflavin-S and PHF-1 signals were increased in cells cotransfected with pALK and pHA-Tau constructs (FIG. 2C). The result demonstrates that ALK increases aggregation and phosphorylation of tau.

### 2-2: CHANGE IN TAU AGGREGATION AND PHOSPHORYLATION DEPENDING ON KINASE ACTIVITY OF HUMAN ALK

To examine whether kinase activity of ALK is needed for tau regulation, the present inventor used various ALK constructs such as wild-type ALK, kinase-inactivated ALK (ALK KD), structurally activated ALK (ALK.Fc), and kinase-inactivated ALK-Fc (ALK.Fc KD) (FIG. 3A). The structurally activated variant ALK.Fc is a chimera protein an extracellular domain of which is substituted with a mouse IgG 2b Fc domain. The Fc domain induces dimerization and oligomerization of the receptor protein leading autophosphorylation and activation of the receptor. ALK KD and ALK.Fc KD proteins, which respectively are kinase-inactivated form of ALK and ALK.Fc, have alternation in unchangeable lysine residue positioned at ATP-binding pocket of a catalytic domain into alanine.

HT22 cells were cotransfected for 24 hours with pGFP-tau construct and one of ALK constructs as shown in FIG. 3A [i.e., wild-type ALK (pALK), the structurally activated variant (pALK.Fc), and the kinase inactivated variant (pALK KD and pALK.Fc KD)]. Aggregation of tau was measured by counting GPF-positive cells appeared to as agglutinated cells. As a result, tau aggregation was increased in HT22 cells in which pALK, pALK.Fc having kinase activity were introduced while tau aggregation of HT22 cells in which pALK KD and pALK.Fc KD having inactivated kinase were introduced showed a similar value to that of a control in which pcDNA was introduced (FIG. 3B). The value shown in the graph was mean ±S.D (n=4), and P-value was calculated by using the t-test pharmaceutical composition. These results demonstrate that kinase activity of ALK is essential for tau aggregation.

Then, the cell extract was analyzed through western blot in reducing and non-reducing conditions. The used antibodies are as follow: PHF-1 (p-Ser396/404; Davies), anti-ALK monoclonal antibody (provided from Dr. Mark Vigny (Souttou et al., J. Biol. Chem., 276: 9526-9531, 2001), CP13 (p-S202; Davies), 12E8 (p-Ser262/356; provided from Dr. Peter Seubert (Elan Pharmaceuticals)), p-ALK (p-Tyr1604; Cell signaling, #3341), and α-tubulin (Sigma, T5168). Consequently, structurally activated ALK.Fc increased expression of anti phosphorylated-ALK, CP12, 12E8, and PHF-1 than wild-type ALK does, while kinase-inactivated ALK KD and ALK.Fc KD variants did not affect at all (FIG. 3C). In addition, ALK.Fc was detected in an oligomer form [dimer: 240 kD, trimer: ∼360 KD] in a non-reducing condition, while wild-type ALK was detected in a monomer form (∼200 kD). Nevertheless, autophosphorylation of ALK and ALK.Fc proteins were detected by anti-phophorylated ALK antibody. Wild-type ALK was less activated than the structurally activated variant ALK.Fc, since dimerization and oligomerization of ALK were facilitated by Fc domain of ALK.Fc thereby increasing kinase activity (see Moog-Lutz et al., J. Biol. Chem., 280: 26039-26048, 2005). These results demonstrate again that kinase activity of ALK plays an important role in tau regulation.

### EXPERIMENTAL EXAMPLE 3: EXAMINATION OF TAU MODIFICATION BY ALK THROUGH ERK AND CDK

### 3-1: EXAMINATION OF INCREASE IN TAU PHOSPHORYLATION BY ALK THROUGH ERK AND CDK IN HT22 CELLS

To investigate which kinase plays a role as a downstream regulator of ALK-induced tau modification, the present inventor coexpressed GFP-tau and ALK or an ALK variant and examined activity of tau kinase. Specifically, pGFP-tau and one of ALK constructs (pALK, pALK KD, pALK.Fc, and pALK.Fc KD) were cotransfected into HT22 cells. As a control, used were a group in which pGFP-tau construct alone was introduced and a group in which pGFP-tau and pcDNA were cotransfected. After 24 hours of transfection, AKT, GSK3*β* and ERK proteins and phosphorylation levels of AKT, GSK3*β* and ERK proteins were examined by using western blot, wherein AKT, GSK3*β* and ERK proteins were known as downstream kinases of ALK in cancer cells. The used antibodies are as follow: AKT (Cell signaling, #9272), p-AKT (Cell signaling, #9271), GSK3*β* (BD Biosciences, 610201), p-GSK3*β* (p-Ser9; Cell signaling, #9323), ERK (Cell signaling, #9102), p-ERK (Cell signaling, #9101), and anti-α-tubulin (Sigma, T5168). Consequently, increases in phosphorylation of AKT and ERK were exhibited only in the pALK.Fc construct having kinase activity and pGFP-tau cotransfected group while that of the GSK3*β* group showed no change (FIG. 4A).

In addition, examined was influence of a tau kinase specific inhibitor on ALK-induced tau phosphorylation. HT22 cells were cotransfected with pGFP-tau construct and a selected pALK.Fc construct, which have a difference in AKT and ERK phosphorylation levels, and then treated with roscovitine (Ros), which is a CKD5 inhibitor, U0126, which is an ERK inhibitor, and LiCl, which is a GSK3β inhibitor, for 24 hours. Thereafter, western bolt was performed using the cell extract. The used antibodies are as follow: PHF-1 (p-Ser396/404; Davies), CP13 (p-S202; Davies), MC-1 (conformational change; Davies), P35/25 (Cell signaling, #2680), TG5 (220-240; generously provided by Dr. Peter Davies, Albert Einstein College of Medicine, NY), -actin (Sigma, A2668), ERK (Cell signaling, #9102), p-ERK (Cell signaling, #9101), GSK3β (BD Biosciences, 610201), and p-GSK3*β* (p-Ser9; Cell signaling, #9323).

Consequently, roscovitine and U0126 significantly reduced ALK-mediated tau phosphorylation in a manner dependent on a treating amount (FIGS. 4B and 4C). In particular, roscovitine showed an excellent inhibiting effect which is better than that of U0126. Specifically, LiCl inhibited tau phosphorylation in both cells expressing pGFP-tau alone and coexpressing pGFP-tau and pALK.Fc, and inhibition levels were very similar (FIG. 4D). These results demonstrate that ALK increases phosphorylation of tau through ERK and CDK activation.

### 3-2: EXAMINATION OF INCREASE IN TAU PHOSPHORYLATION BY ALK IN TAU-TRANSFORMED HT22 CELL LINE

Since substantially no tau was expressed in most neuronal cells, the present inventor reexamined influence of ALK on tau aggregation and phosphorylation by using the tau-transformed HT22 cell line produced in Example 1-2 which stably expressing tau.

HT22/Tau #1 and #3 clones, which were HT22/Tau transformed cell lines, were cultured for 24 hours with/without 1 µg/ml doxycycline (Dox) treatment. The cultured cells were extracted to measure tau protein expression through western blot. Consequently, tau expression was induced when tau expression was induced through doxycycline treatment in HT22/Tau mixed cells produced in an example of the present invention, and in monoclones #1 and #3 which were extracted from the mixed cells. Also, an observed tau expression level of HT22/Tau #1 cells was lower than that of HT22/Tau #3 cells (FIG. 5A). As a control, HT22/Hph cells produced by transducing pBig2i-tau (tet-on) were used.

Subsequently, the present inventor performed cotransfection on a HT22/Tau #1 transformed cell line with pcDNA (a control), pALK KD, or pALK.Fc, and cultured the cells for 24 hours with/without 1 µg/ml Doxycycline (Dox) treatment. Then, the cell extract was analyzed by using western blot. As shown in FIG. 5B, ALK in an active state, not ALK KD, increased tau phosphorylation. The result corresponds to the result obtained by transiently overexpressing ALK.Fc in HT22 cells in Experiment Examples 2 and 3.

Subsequently, the present inventor performed transfection on a HT22/Tau #1 transformed cell line with pALK.Fc and pcDNA (a control) or pCDK5 DN (a CDK5 dominant-negative variant; D144N), and cultured the cells for 24 hours with/without 1 µg/ml doxycycline (Dox) treatment. Then, the cells were extracted to perform western blot. Consequently, ALK-induced tau phosphorylation was decreased by inhibiting endogenous CKD5 activation through the CDK5 dominant-negative (CDK5 DN) variant (FIG. 5C). The result demonstrates that CDK5 activity is needed for tau regulation by ALK, and it can be found that the tau-transformed HT22 cell line which stably expressing tau is also exhibited the same result as observed in the Experimental Examples 1 and 2.

### EXPERIMENTAL EXAMPLE 4: EXAMINATION OF INCREASE IN TAU PHOSPHORYLATION BY ALK

### 4-1: INCREASE IN TAU PHOSPHORYLATION BY ALK AGONISTIC ANTIBODY IN MOUSE PRIMARY CELLS

Since a definite natural ligand for ALK has been not known, the present inventor used overexpression analysis to examine that ALK activation increases phosphorylation and aggregation of tau in Experimental Example. Then, to examine whether endogenous ALK activation has the similar effect on tau, the present inventor performed the experiment in primary cells, which was obtained by directly culturing neuronal cells of mouse brain tissue, using antibodies showing agonistic and antagonistic action to ALK.

At first, an ALK expression level was examined in a mouse brain. Mouse brain tissue was dissected portion-wise. Then, brain tissue was homogenized using a TBS buffer [20 mM Tris-Cl (pH 7.4), 150 mM NaCl, 1% Triton X-100, 1 mM Na₃VO4, 1 mM NaF, 1 mM PMSF, and 1 µg/ml of aprotinin, leupeptin and pepstatin A]. The homogenate was centrifuged at 15,000 g for 30 minutes, and thereafter a protein concentration of obtained supernatant was measured by using Bradford assay (Bio-Rad). Through western blot assay, it was examined that ALK was expressed in various portion of the mouse brain such as cerebellar cortex, hippocampus, striatum, and brainstem on postnatal day 1 (PI) (FIG. 6A). ALK expression was relatively higher in cerebellar cortex.

Subsequently, ALK expression was examined in mouse primary neuronal cells prepared by isolating and then culturing mouse brain tissue. Oligodendrocyte and neuronal cells were isolated from a hippocampus of a PI mouse through Optiprep concentration gradient. Thereafter, ALK expression of the mouse was observed through western blot using anti-ALK antibody. Also, ALK expression was examined in neuronal cells (FIG. 6B).

### 4-2: EXAMINATION OF INCREASE IN TAU PHOSPHORYLATION BY ALK AGONISTIC ANTIBODY IN MOUSE PRIMARY CELLS

Moreover, specificity of mAb46 and mAb30 monoclonal antibodies was examined, wherein the mAb46 monoclonal antibody has the agonistic effect to simulate endogenous ALK and the mAb30 monoclonal antibody has the antagonistic effect to an extracellular domain of ALK. HT22 cells were cotransfected with pcDNA (a control) or pALK construct then cultured for 24 hours to obtain cell extract. Western blot was performed on the cell extract by respectively using mAb46 showing the agonistic effect and mAb30 showing the antagonistic effect. The mAb46 and mAb30 antibodies were provided from Dr. Mel Vigny (Souttou et al., J. Biol. Chem., 276: 9526-9531, 2001). Consequently, it was examined that the monoclonal antibody was not react to ALK-nonspecific protein (FIG. 6C).

Then, mouse primary cells were treated with mAb46 and mAb30 antibodies to examine agonistic and antagonistic effects. Cells were pretreated with 6 nM mAb30 antibody for one hours, and then respectively treated with mAb46 (mAb30 -> mAb46), or 6 nM Ab46. For negative control, cells treated with IgG alone were used, and for positive control, 1 µg of bovine serum albumin (BSA) was used. The amount of the used monoclonal antibody was examined by using coomassie blue staining. Western blot was performed using PHF-1 and anti-tau antibodies, and the relative ratio of PHF-1 and Tau-5 signals (PHF-1/Tau5) was numerically indicated in the bottom panel. Consequently, it was examined that tau phosphorylation was increased as PHF expression increased when mAb46, which has the agonistic effect on primary cerebral cortex neuronal cells, was treated (FIG. 6D bottom). In contrast, it was examined that mAb46-mediated tau phosphorylation was inhibited when mAb46 having the agonistic effect was treated after reacting with mAb30 having the antagonistic effect (FIG. 6D bottom). These results demonstrate that mAb46 and mAb30 antibodies effectively show the agonistic and antagonistic actions on ALK, and ALK has the effect of increasing tau phosphorylation.

### 4-3: EXAMINATION OF INCREASE IN TAU PHOSPHORYLATION BY mAb46 ANTIBODY IN HT22 CELLS

Firstly, endogenous ALK expression was examined in a membrane fraction of HT22 cells. HT22 cells was dissolved by reacting with a homogenization buffer [10 mM HEPES (pH 7.4), 250 mM sucrose, 1 mM EDTA, 1 mM EGTA, 1 mM Na₃VO4, 1 mM NaF, 1 mM PMSF, 1 µg/ml aprotinin, 1 µg/ml leupeptin and 1 µ g/ml pepstatin A] and then repetitively moving a microinjection needle in up-down motion. Undisrupted cells and nucleus were separated through centrifugation for 10 minutes under 4°C and 1500 g condition, and then the supernatant was centrifuged again for 2 hours under 4°C and 100,000 g condition. Consequently, obtained pellet includes membrane fraction while the supernatant includes cytoplasmic fraction. Then, the fraction was analyzed through western blot using anti-ALK antibody. Consequently, it was examined that ALK was expressed in HT22 cell membrane fraction (FIG. 7A).

In addition, the present inventor examined influence of mAb46 on tau phosphorylation in HT22 neuronal cells. HT22 cells were transfected with pGFP-tau construct, and treated with anti-ALK antibody. A phosphorylation level of tau was examined through western blot, and tau aggregation was observed under fluorescence microscopy. Consequently, as the same as the observed result of primary neuronal cells directly isolated from a mouse brain and then cultured, mAB46 treatment increased phosphorylation and aggregation of HT22/tau cells, while mAb30, which has the antagonistic effect, decreased the effect of mAb46 to facilitate tau phosphorylation (FIGS 7B and 7C). These results demonstrate that intracellular tau phosphorylation was induced by endogenous ALK stimulation or activation.

### EXPERIMENTAL EXAMPLE 5: EXAMINATION OF INCREASE IN TAU-MEDIATED NEUROTOXICITY BY ALK

### 5-1: EXAMINATION OF NEURONAL CELL-SPECIFIC CELL DEATH BY ALK/TAU CYTOTOXICITY IN NEURONAL CELLS

Examined was a cell death level of neuronal cells containing a lot of aggregated and hyperphosphorylated tau due to ALK. HT22 cells were cotransfected with pALK.Fc or pALK.Fc KD construct and pGFP or pGFP-tau construct, and cultured for 24 to 96 hours. Cell death was measured by staining cells with ethidium homodimer (EtHD) and then counting GFP-positive cells indicating condensed and fragmented nuclei. As a result, ectopic expression of tau in HT22 cells caused toxicity on neuronal cells thereby causing cell death at a high level, while ALK.Fc caused low level of cell death than tau did (FIG. 8A). It was noted that ectopic expression of structurally activated ALK.Fc increased cell death in neuronal cells expressing tau, while ALK.Fc KD having inactivated kinase did not affect on cell death (FIG. 8A). These results demonstrate that ALK activation enhance toxicity of tau in neuronal cells.

### 5-2: EXAMINATION OF DOWNSTREAM KINASE OF ALK/TAU CYTOTOXICITY IN NEURONAL CELLS

To examine downstream kinase of ALK-mediated tau neurotoxicity, the present inventor used a kinase inhibitor. HT22 cells were cotransfected with pALK.Fc and pGFP-tau constructs, and then treated with mock (Mock), 25 µM roscovitine (Ros), 1 µM U0126 (U0126), or 10 mM LiCl for 24 hours. Thereafter, cell death was measured. For negative control, used was mock group in which none of a material was treated. Consequently, it was observed that ALK.Fc/Tau-mediated neurotoxicity was significantly reduced by roscovitine treatment, and partially reduced by U0126 treatment (FIG. 8B).

Subsequently, to examine downstream kinase of ALK-mediated tau neurotoxicity, the present inventor used a dominant-negative variant of kinase. HT22 cells were cotransfected with pGFP-tau, and pALK.Fc together with one construct among pcDNA, pMEK1 DN, pAKT1 DN, pp38 DN, pJNK DN, or pCDK5 DN construct. After 48 hours of the transfection, cell death was measured. As a result, cell death was inhibited in HT22 cells cotransfected with pMEK1 DN and pCDK5 DN constructs. It means that ALK.Fc/Tau-mediated cell death was inhibited by expression of a CDK5 or ERK dominant-negative variant (FIG. 8C).

Moreover, the present inventor observed neurotoxicity of ALK/Tau by using CDK5 shRNA. HT22 cells cotransfected with pGFP-tau and pALK.Fc constructs together with either pCDK5 shRNA #1 or #2. After 48 hours of the transfection, cell death was measured by using LIVE/DEAD Viability/Cytotoxicity kit (Molecular Probes). As the same as above experimental results, it was observed that cell death induced by ALK/Tau was reduced when endogenous CDK5 expression was inhibited using shRNA (FIG. 8D). The used pCDK5 shRNA #1 was characterized by having a polynucleotide having the nucleotide sequence of SEQ ID No. 1 (5'-TGACCAAGCTGCCAGACTA-3'), and pCDK5 shRNA #2 was characterized by having a polynucleotide having the nucleotide sequence of SEQ ID No. 2 (5'-ACTCCACGTCCATCGACAT-3'). These results demonstrate that CDK5 and ERK are needed for ALK-induced tau toxicity in neuronal cells. The results shown in FIGS 8A to 8D were mean ± S.D obtained by triplicate experiments.

### 5-3: EXAMINATION OF ALK/TAU CYTOTOXICITY IN NON-NEURONAL CELLS

To examine whether ALK-activated tau triggers cell death in non-neuronal cells, the present inventor transfected MCF7 cells with pGFP-tau together with one construct among pALK, pALK KD, pALK.Fc, or pCaspase-8 (a positive control). After 48 hours of the transfection, cell death was measured by EtHD staining, and the cell extract was analyzed through western blot. Consequently, although phosphorylation of ALK and ERK was observed, cell death was not increased (FIGS 8D and 8E). The result demonstrates that ALK plays a role in exhibiting cytotoxicity in a neuronal cell-specific manner.

### 5-4: EXAMINATION OF INHIBITION IN CELL DEATH BY ALK/TAU THROUGH CDK5 INHIBITOR TREATMENT

HT22 cells were cotransfected with pALK.Fc or pALK.Fc KD and pGFP or pGFPtau, and 24hours after then, a cell image was obtained with a fluorescence microscopy. In contrast to the study result by Motegi *et al.,* in that ALK activity activates neurite growth in neuronal cell and fruitfly models (Motegi et al., J. Cell Sci., 117, 3319-3329m, 2004), the present inventor observed that no neurite growth facilitation was induced by ALK.Fc when pALK.Fc and pGFP-tau were coexpressed (FIG. 9A).

A HT22/Tau #1 transformed cell line was cotransfected with pGFP and pcDNA or pALK after pretreated with 25 µM of roscovitine. Thereafter, the resultant was cultured for 24 hours with/without doxycycline (Dox) treatment. Neurite growth and cell viability were measured through a fluorescence microscopy. Relative ratio of neurites was measured by the number of neuronal cells verses the length of neurites. Consequently, the reduction in neurite growth and the increase in cell death by ALK and tau in neuronal cells expressing tau and ALK.Fc were recovered through the CDK5 inhibitor, roscovitine, treatment (FIG. 9B). These results mean that CDK5 plays an important role in ALK-mediated neurite growth and neurotoxicity.

### EXPERIMENTAL EXAMPLE 6: EXAMINATION OF INHIBITION IN HYPERPHOSPHORYLATION AND CYTOTOXICITY OF TAU BY ALK-SPECIFIC INHIBITOR TREATMENT

The present inventor tired to examine whether an ALK-specific inhibitor directly inhibits tau phosphorylation and tau toxicity facilitation induced by activated ALK. Used were two types of ALK inhibitors, NVP-TAE684 (Novartis) and PF-2341066 (Pfizer) which were known to have excellent specificity to ALK (see Galkin et al., Proc. Natl. Acad. Sci. USA, 104, 270-275, 2007; Zou et al., Cancer Res., 67, 4408-4417, 2007). Specifically, HT22/Tau #1 cells were cotransfected with pGFP and pcDNA or pALK.Fc, and then cultured under the condition that 1 µg/ml of doxycycline (Dox) was treated/untreated while increasing a concentration of treated ALK inhibitor (NVP-TAE684, PF-2341066) for 24 hours. Then, cell death was determined through a fluorescence microscopy based on cell morphology. Thereafter, cells were extracted and phosphorylation of tau, ERK, CDK5 and ALK was measured through western blot using respective antibodies. The error bar of the graph indicates mean ±S.D. (n=4).

Consequently, it was observed that cell death was significantly reduced when HT22/Tau #1 cells were transfected with ALK.Fc and then treated with an ALK inhibitor. Specifically, cell death was decreased from 42% to 10% by 20 nM NVP-TAE684 treatment, and cell death was decreased from 42% to 15% by 100 nM PF-2341066 treatment (FIG. 10A). Moreover, in the western blot result using cells extracted after observing with a fluorescence microscopy, it was observed that ALK tyrosine phosphorylation was completely inhibited by NVP-TAE684 and partially inhibited by PF-2341066 (FIG. 10B). Also, both ALK-mediated ERK activation and tau phosphorylation were inhibited in a manner dependent on an amount of treated ALK (FIG. 10B). These results demonstrate that ALK kinase activity is needed for both tau phosphorylation and tau-mediated neurotoxicity.

### EXPERIMENTAL EXAMPLE 7: EXAMINATION OF INCREASE IN TAU-MEDIATED NEURONAL DEGENERATION BY INCREASED ALK ACTIVITY IN TAU FRUITFLY MODEL

### 7-1: OBSERVATION OF CHANGE IN EYE PHENOTYPE BY INCREASED ALK ACTIVITY IN TAU FRUITFLY MODEL

To measure the *in vivo* effect of ALK to regulate tau phosphorylation and toxicity, the present inventor used human tau-transformed *Diptera* (gl-tau2.1 line) which has been well known as a fruitfly model for tauopathy. In a gl-tau2.1 line fruitfly, human tau is expressed in photoreceptor neuronal cells. The gl-tau fruitfly showed neuronal degeneration, abnormal ommatidia and hair, and as well as minor disruption in a retina compared to a wild-type control fruitfly. A transgene was expressed by using a binary GAL4/UAS system (Brand and Perrimon, 1993). It is possible to express transgene in a cell group around tau-expressing cells by using the system.

FIG. 11A is a microscopic image showing that rough eye phenotype and irregular eye shapes were increased when structurally activated fruitfly ALK was coexpressed in tau-transformed fruitfly. Experimental groups are as follows: a control (GMR-GAL4/+), dALKACT (GMR-GAL4/+; UAS-dALKACT/+), dALKDN (GMR-GAL4/+;UAS-dALKDN/+), gl-Tau (GMRGAL4/+; gl-Tau2.1/+), gl-Tau/dALKACT (GMR-GAL4/+; gl-Tau2.1/+; UASdALKACT/+), and gl-Tau/dALKDN (GMR-GAL4/+; gl-Tau2.1/+; UAS-dALKDN/+).

Consequently, transformed fruitfly (Tau/dALK^{ACT}) simultaneously expressing structurally active fruitfly ALK and tau showed a reduced size and more rough shapes than a fruitfly expressing a tau gene alone. In contrast, when the gl-tau transformed fruitfly was crossed with a dominant-negative ALK fruitfly (Tau/dALK^{DN}), rough eye phonotype was significantly reduced (FIG. 11A). Normal eye phenotype observed by using GMR-GAL4 driver was inhibited when fruitfly ALK in an activated form (dALK^{ACT}) was overexpressed in eyes (FIG. 11A).

### 7-2: EXAMINATION OF INNER NEURONAL DEGENERATION BY INCREASE IN ALK ACTIVITY IN TAU FRUITFLY MODEL

Subsequently, an inner change in neuronal degeneration was observed with fluorescence microscopy when ALK was coexpressed in a tau-transformed fruitfly. On day 5, an image of an inner retina of a fruitfly was taken through a fluorescence microscopy after immunostaining a nucleus with Hoechst 33342. The scale bar shown in FIG. 11B means 10 µm. Then, the thickness of the retina was digitized based on a taken image. Consequently, it was observed that the thickness of a retina of a Tau/dALK^{ACT} transformed fruitfly was significantly reduced and an inner structure of a retina was disrupted due to the severe injury in nerves (FIGS 11B and 11C). The error bar shown FIG. 11C indicates mean ± S.E.M (n = 3).

Since tau toxicity was dependent on phosphorylation of tau, it was examined that a tau phosphorylation level of the transformed tau fruitfly was regulated by dALK^{ACT} and dALK^{DN} through western blot. Fruitfly head tissue was homogenized using a homogenizing buffer [50 mM Tris-Cl (pH 8.0), 150 mM NaCl, 1% Triton X-100, 10% sucrose, 1 mM Na₃VO4, 1 mM NaF, 1 mM PMSF, and 1 µg/ml of aprotinin, leupeptin and pepstatin A]. The homogenate was centrifuged at 15,000 g for 30 minutes, and thereafter a protein concentration of the obtained supernatant was measured by using the Bradford assay (Bio-Rad). The obtained sample was used for western blot experiment. Consequently, as shown in FIG. 11D, it was observed that PHF-1 staining became stronger when structurally active fruitfly ALK was coexpressed with tau than a single gene of tau was overexpressed in a fruitfly. In contrast, almost no PHF-1 staining was observed in a fruitfly in which dominant-negative frutifly ALK and tau were overexpressed (FIG. 11D). Taken together, these results demonstrate that ALK activity increases neurotoxicity in tau-transformed fruitfly *in vivo.*

Although the present invention has been described with reference to the drawing and the described example, and example for only exemplary purpose, it should be understood that numerous alternation and other equivalent examples and experimental examples may be devised by a person skilled in the art. Therefore, the technical protection scope of the present invention should be determined based on the following claims.

### SEQUENCE LISTING FREE TEXT

SEQ ID NO. 1 represents pCDK5 shRNA #1.
SEQ ID NO. 2 represents pCDK5 shRNA #2.

## Claims

1. A pharmaceutical composition for use in treating
(i) tau protein-mediated Alzheimer's disease in which tau protein is hyper-phosphorylated or aggregated or in which there is tau protein neurotoxicity, or
(ii) tau protein-mediated frontotemporal lobar degeneration in which tau protein is hyper-phosphorylated or aggregated or in which there is tau protein neurotoxicity,
said composition comprising a therapeutically effective amount of an inhibitor of the dimerization, the activation or the expression of anaplastic lymphoma kinase (ALK).

2. The pharmaceutical composition for use according to claim 1, wherein the inhibitor is an antagonizing antibody to ALK or a functional derivative thereof, an ALK-specific inhibiting compound, an anti-sense nucleotide, siRNA, shRNA, or miRNA against an ALK gene.

3. The pharmaceutical composition for use according to claim 1, wherein the inhibitor is an ALK kinase activity inhibitor.

4. A method for screening a drug for treating
(i) tau protein-mediated Alzheimer's disease in which tau protein is hyper-phosphorylated or aggregated or in which there is tau protein neurotoxicity, or
(ii) tau protein-mediated frontotemporal lobar degeneration in which tau protein is hyper-phosphorylated or aggregated or in which there is tau protein neurotoxicity,
comprising:
treating a test compound to a reaction solution including ALK, ATP, and a substrate of ALK;
measuring a change in a phosphorylation level of the substrate of ALK; and
selecting a test compound which significantly inhibits phosphorylation of the substrate of ALK compared to that of a negative control which is not treated with the test compound.

5. An *in vitro* method for screening a drug for treating
(i) tau protein-mediated Alzheimer's disease in which tau protein is hyper-phosphorylated or aggregated or in which there is tau protein neurotoxicity, or
(ii) tau protein-mediated frontotemporal lobar degeneration in which tau protein is hyper-phosphorylated or aggregated or in which there is tau protein neurotoxicity,
comprising:
examining the phosphorylation of a substrate of ALK in cells treated with the test compound; and
selecting a test compound which inhibits the phosphorylation of the substrate of ALK compared to a negative control which is not treated with the test compound.

6. A method for screening a drug for treating
(i) tau protein-mediated Alzheimer's disease in which tau protein is hyper-phosphorylated or aggregated or in which there is tau protein neurotoxicity, or
(ii) tau protein-mediated frontotemporal lobar degeneration in which tau protein is hyper-phosphorylated or aggregated or in which there is tau protein neurotoxicity,
comprising:
treating a test compound with a reaction solution including ALK, ATP, and a substrate of ALK; and
selecting a test compound which inhibits an interaction between ALK and the substrate of ALK compared to a negative control which is not treated with the test compound.

7. An *in vitro* method for screening a drug for treating
(i) tau protein-mediated Alzheimer's disease in which tau protein is hyper-phosphorylated or aggregated or in which there is tau protein neurotoxicity, or
(ii) tau protein-mediated frontotemporal lobar degeneration in which tau protein is hyper-phosphorylated or aggregated or in which there is tau protein neurotoxicity,
comprising:
treating cells expressing ALK with a test compound and a substrate of ALK; and
selecting a test compound which inhibits an interaction between ALK and the substrate of ALK compared to a negative control which is not treated with the test compound.

8. A method for screening a drug for treating
(i) tau protein-mediated Alzheimer's disease in which tau protein is hyper-phosphorylated or aggregated or in which there is tau protein neurotoxicity, or
(ii) tau protein-mediated frontotemporal lobar degeneration in which tau protein is hyper-phosphorylated or aggregated or in which there is tau protein neurotoxicity,
comprising:
treating a test compound with a reaction solution including ALK, and a ligand of ALK; and
selecting a test compound which inhibits an interaction between ALK and the ligand of ALK compared to a negative control which is not treated with the test compound.

9. An *in vitro* method for screening a drug for treating
(i) tau protein-mediated Alzheimer's disease in which tau protein is hyper-phosphorylated or aggregated or in which there is tau protein neurotoxicity, or
(ii) tau protein-mediated frontotemporal lobar degeneration in which tau protein is hyper-phosphorylated or aggregated or in which there is tau protein neurotoxicity,
comprising:
treating cells expressing ALK with a ligand of ALK and a test compound; and
selecting a test compound which inhibits an interaction between ALK and the ligand of ALK compared to a negative control which is not treated with the test compound.

10. An *in vitro* method for screening a drug for treating
(i) tau protein-mediated Alzheimer's disease in which tau protein is hyper-phosphorylated or aggregated or in which there is tau protein neurotoxicity, or
(ii) tau protein-mediated frontotemporal lobar degeneration in which tau protein is hyper-phosphorylated or aggregated or in which there is tau protein neurotoxicity,
comprising:
treating cells expressing ALK with a test compound;
measuring a change in a phosphorylation level of cyclin-dependent kinase-5 (CDK-5) or extracellular signal-regulated kinase (ERK) in cells treated with the test compound; and
selecting a test compound which significantly inhibits the phosphorylation of CDK5 or ERK compared to a negative control untreated with the test compound.

11. A method for screening a drug for treating
(i) tau protein-mediated Alzheimer's disease in which tau protein is hyper-phosphorylated or aggregated or in which there is tau protein neurotoxicity, or
(ii) tau protein-mediated frontotemporal lobar degeneration in which tau protein is hyper-phosphorylated or aggregated or in which there is tau protein neurotoxicity,
comprising:
treating a transformed fruitfly expressing ALK and tau (Tau/dALK) with a test compound;
observing eye phenotype or neuronal degeneration by measuring a shape of an ommatidium or a change in a retinal thickness of the transformed fruitfly treated with the test compound; and
selecting a test compound which significantly inhibits a change in a ommatidium shape or disruption of a retina compared to a negative control untreated with the test compound.

12. A method for screening a drug for treating
(i) tau protein-mediated Alzheimer's disease in which tau protein is hyper-phosphorylated or aggregated or in which there is tau protein neurotoxicity, or
(ii) tau protein-mediated frontotemporal lobar degeneration in which tau protein is hyper-phosphorylated or aggregated or in which there is tau protein neurotoxicity,
comprising:
treating ALK protein with a test compound;
observing whether the ALK protein forms a dimer or not; and
selecting a test compound which inhibits the dimer formation of ALK protein.

13. The method according to claims 8 or 9, wherein the ALK ligand may be pleiotrophin (PTN), midkine (MK) or Jelly belly (Jeb).

## Patentansprüche

1. Pharmazeutische Zusammensetzung zur Verwendung beim Behandeln von
(i) Tau-Protein-vermittelter Alzheimer-Krankheit, bei der Tau-Protein hyperphosphoryliert oder aggregiert ist oder bei der Tau-Protein-Neurotoxizität vorliegt, oder
(ii) Tau-Protein-vermittelter frontotemporaler Lobärdegeneration, bei der Tau-Protein hyperphosphoryliert oder aggregiert ist oder bei der Tau-Protein-Neurotoxizität vorliegt,
wobei die Zusammensetzung eine therapeutisch wirksame Menge eines Inhibitors der Dimerisierung, der Aktivierung oder der Expression von anaplastischer Lymphomkinase (ALK) umfasst.

2. Pharmazeutische Zusammensetzung zur Verwendung nach Anspruch 1, wobei der Inhibitor ein antagonisierender Antikörper gegen ALK oder ein funktionelles Derivat davon, eine ALK-spezifische hemmende Verbindung, ein Antisens-Nukleotid, siRNA, shRNA oder miRNA gegen ein ALK-Gen ist.

3. Pharmazeutische Zusammensetzung zur Verwendung nach Anspruch 1, wobei der Inhibitor ein ALK-Kinase-Aktivitätsinhibitor ist.

4. Verfahren zum Screening eines Arzneimittels zum Behandeln von
(i) Tau-Protein-vermittelter Alzheimer-Krankheit, bei der Tau-Protein hyperphosphoryliert oder aggregiert ist oder bei der Tau-Protein-Neurotoxizität vorliegt, oder
(ii) Tau-Protein-vermittelter frontotemporaler Lobärdegeneration, bei der Tau-Protein hyperphosphoryliert oder aggregiert ist oder bei der Tau-Protein-Neurotoxizität vorliegt,
umfassend:
Behandeln einer Testverbindung mit einer Reaktionslösung, die ALK, ATP und ein Substrat von ALK beinhaltet;
Messen einer Änderung des Phosphorylierungsniveaus des Substrats von ALK; und
Auswählen einer Testverbindung, die die Phosphorylierung des Substrats von ALK im Vergleich zu einer negativen Kontrolle, die nicht mit der Testverbindung behandelt wird, signifikant hemmt.

5. *In*-*vitro*-Verfahren zum Screening eines Arzneimittels zum Behandeln von
(i) Tau-Protein-vermittelter Alzheimer-Krankheit, bei der Tau-Protein hyperphosphoryliert oder aggregiert ist oder bei der Tau-Protein-Neurotoxizität vorliegt, oder
(ii) Tau-Protein-vermittelter frontotemporaler Lobärdegeneration, bei der Tau-Protein hyperphosphoryliert oder aggregiert ist oder bei der Tau-Protein-Neurotoxizität vorliegt,
umfassend:
Untersuchen der Phosphorylierung eines Substrats von ALK in Zellen, die mit der Testverbindung behandelt wurden; und
Auswählen einer Testverbindung, die die Phosphorylierung des Substrats von ALK im Vergleich zu einer negativen Kontrolle, die nicht mit der Testverbindung behandelt wird, hemmt.

6. Verfahren zum Screening eines Arzneimittels zum Behandeln von
(i) Tau-Protein-vermittelter Alzheimer-Krankheit, bei der Tau-Protein hyperphosphoryliert oder aggregiert ist oder bei der Tau-Protein-Neurotoxizität vorliegt, oder
(ii) Tau-Protein-vermittelter frontotemporaler Lobärdegeneration, bei der Tau-Protein hyperphosphoryliert oder aggregiert ist oder bei der Tau-Protein-Neurotoxizität vorliegt,
umfassend:
Behandeln einer Testverbindung mit einer Reaktionslösung, die ALK, ATP und ein Substrat von ALK beinhaltet; und
Auswählen einer Testverbindung, die eine Wechselwirkung zwischen ALK und dem Substrat von ALK im Vergleich zu einer negativen Kontrolle, die nicht mit der Testverbindung behandelt wird, hemmt.

7. *In*-*vitro*-Verfahren zum Screening eines Arzneimittels zum Behandeln von
(i) Tau-Protein-vermittelter Alzheimer-Krankheit, bei der Tau-Protein hyperphosphoryliert oder aggregiert ist oder bei der Tau-Protein-Neurotoxizität vorliegt, oder
(ii) Tau-Protein-vermittelter frontotemporaler Lobärdegeneration, bei der Tau-Protein hyperphosphoryliert oder aggregiert ist oder bei der Tau-Protein-Neurotoxizität vorliegt,
umfassend:
Behandeln von ALK-exprimierenden Zellen mit einer Testverbindung und einem Substrat von ALK; und
Auswählen einer Testverbindung, die eine Wechselwirkung zwischen ALK und dem Substrat von ALK im Vergleich zu einer negativen Kontrolle, die nicht mit der Testverbindung behandelt wird, hemmt.

8. Verfahren zum Screening eines Arzneimittels zum Behandeln von
(i) Tau-Protein-vermittelter Alzheimer-Krankheit, bei der Tau-Protein hyperphosphoryliert oder aggregiert ist oder bei der Tau-Protein-Neurotoxizität vorliegt, oder
(ii) Tau-Protein-vermittelter frontotemporaler Lobärdegeneration, bei der Tau-Protein hyperphosphoryliert oder aggregiert ist oder bei der Tau-Protein-Neurotoxizität vorliegt,
umfassend:
Behandeln einer Testverbindung mit einer Reaktionslösung, die ALK und einen Liganden von ALK beinhaltet; und
Auswählen einer Testverbindung, die eine Wechselwirkung zwischen ALK und dem Liganden von ALK im Vergleich zu einer negativen Kontrolle, die nicht mit der Testverbindung behandelt wird, hemmt.

9. *In*-*vitro*-Verfahren zum Screening eines Arzneimittels zum Behandeln von
(i) Tau-Protein-vermittelter Alzheimer-Krankheit, bei der Tau-Protein hyperphosphoryliert oder aggregiert ist oder bei der Tau-Protein-Neurotoxizität vorliegt, oder
(ii) Tau-Protein-vermittelter frontotemporaler Lobärdegeneration, bei der Tau-Protein hyperphosphoryliert oder aggregiert ist oder bei der Tau-Protein-Neurotoxizität vorliegt,
umfassend:
Behandeln von ALK-exprimierenden Zellen mit einem Liganden von ALK und einer Testverbindung; und
Auswählen einer Testverbindung, die eine Wechselwirkung zwischen ALK und dem Liganden von ALK im Vergleich zu einer negativen Kontrolle, die nicht mit der Testverbindung behandelt wird, hemmt.

10. *In*-*vitro*-Verfahren zum Screening eines Arzneimittels zum Behandeln von
(i) Tau-Protein-vermittelter Alzheimer-Krankheit, bei der Tau-Protein hyperphosphoryliert oder aggregiert ist oder bei der Tau-Protein-Neurotoxizität vorliegt, oder
(ii) Tau-Protein-vermittelter frontotemporaler Lobärdegeneration, bei der Tau-Protein hyperphosphoryliert oder aggregiert ist oder bei der Tau-Protein-Neurotoxizität vorliegt,
umfassend:
Behandeln von ALK-exprimierenden Zellen mit einer Testverbindung;
Messen einer Änderung in einem Phosphorylierungsniveau von cyclinabhängiger Kinase-5 (CDK-5) oder extrazellulärer signalregulierter Kinase (ERK) in mit der Testverbindung behandelten Zellen; und
Auswählen einer Testverbindung, die die Phosphorylierung von CDK5 oder ERK im Vergleich zu einer negativen Kontrolle, die nicht mit der Testverbindung behandelt wird, signifikant hemmt.

11. Verfahren zum Screening eines Arzneimittels zum Behandeln von
(i) Tau-Protein-vermittelter Alzheimer-Krankheit, bei der Tau-Protein hyperphosphoryliert oder aggregiert ist oder bei der Tau-Protein-Neurotoxizität vorliegt, oder
(ii) Tau-Protein-vermittelter frontotemporaler Lobärdegeneration, bei der Tau-Protein hyperphosphoryliert oder aggregiert ist oder bei der Tau-Protein-Neurotoxizität vorliegt,
umfassend:
Behandeln einer transformierten Fruchtfliege, die ALK und Tau (Tau/dALK) exprimiert, mit einer Testverbindung;
Beobachten des Augenphänotyps oder der neuronalen Degeneration durch Messen einer Form eines Ommatidiums oder einer Änderung einer Netzhautdicke der transformierten Fruchtfliege, die mit der Testverbindung behandelt wurde; und
Auswählen einer Testverbindung, die eine Änderung einer Ommatidiumform oder Beeinträchtigung einer Netzhaut im Vergleich zu einer negativen Kontrolle, die nicht mit der Testverbindung behandelt wird, signifikant hemmt.

12. Verfahren zum Screening eines Arzneimittels zum Behandeln von
(i) Tau-Protein-vermittelter Alzheimer-Krankheit, bei der Tau-Protein hyperphosphoryliert oder aggregiert ist oder bei der Tau-Protein-Neurotoxizität vorliegt, oder
(ii) Tau-Protein-vermittelter frontotemporaler Lobärdegeneration, bei der Tau-Protein hyperphosphoryliert oder aggregiert ist oder bei der Tau-Protein-Neurotoxizität vorliegt,
umfassend:
Behandeln von ALK-Protein mit einer Testverbindung;
Beobachten, ob das ALK-Protein ein Dimer bildet oder nicht; und
Auswählen einer Testverbindung, die die Dimer-Bildung von ALK-Protein hemmt.

13. Verfahren nach den Ansprüchen 8 oder 9, wobei der ALK-Ligand Pleiotrophin (PTN), Midkin (MK) oder Jelly belly (Jeb) sein kann.

## Revendications

1. Composition pharmaceutique pour utilisation dans le traitement des affections suivantes :
(i) la maladie d'Alzheimer médiée par la protéine tau, dans laquelle la protéine tau est hyperphosphorylée ou agrégée ou dans laquelle il y a une neurotoxicité de la protéine tau, ou
(ii) une dégénérescence du lobe frontotemporel médiée par la protéine tau, dans laquelle la protéine tau est hyperphosphorylée ou agrégée ou dans laquelle il y a une neurotoxicité de la protéine tau,
ladite composition comprenant une quantité thérapeutiquement efficace d'un inhibiteur de la dimérisation, de l'activation ou de l'expression de la kinase du lymphome anaplasique (ALK).

2. Composition pharmaceutique pour utilisation selon la revendication 1, dans laquelle l'inhibiteur est un anticorps antagoniste à l'ALK ou à un dérivé fonctionnel de celui-ci, un composé inhibiteur spécifique à l'ALK, un nucléotide antisens, siARN, shARN ou miARN contre un gène d'ALK.

3. Composition pharmaceutique pour utilisation selon la revendication 1, dans laquelle l'inhibiteur est un inhibiteur de l'activité de la kinase ALK.

4. Procédé de criblage d'un médicament pour traiter :
(i) la maladie d'Alzheimer médiée par la protéine tau, dans laquelle la protéine tau est hyperphosphorylée ou agrégée ou dans laquelle il y a une neurotoxicité de la protéine tau, ou
(ii) une dégénérescence du lobe frontotemporel médiée par la protéine tau, dans laquelle la protéine tau est hyperphosphorylée ou agrégée ou dans laquelle il y a une neurotoxicité de la protéine tau,
comprenant :
le traitement d'un composé d'essai à une solution réactionnelle incluant l'ALK, l'ATP et un substrat d'ALK.
la mesure d'un changement du niveau de phosphorylation du substrat d'ALK ; et
la sélection d'un composé d'essai qui inhibe de manière significative la phosphorylation du substrat d'ALK en comparaison de celle d'un témoin négatif qui n'est pas traité avec le composé d'essai.

5. Procédé *in vitro* pour cribler un médicament pour traiter :
(i) la maladie d'Alzheimer médiée par la protéine tau, dans laquelle la protéine tau est hyperphosphorylée ou agrégée ou dans laquelle il y a une neurotoxicité de la protéine tau, ou
(ii) une dégénérescence du lobe frontotemporel médiée par la protéine tau, dans laquelle la protéine tau est hyperphosphorylée ou agrégée ou dans laquelle il y a une neurotoxicité de la protéine tau,
comprenant :
l'examen de la phosphorylation d'un substrat d'ALK dans des cellules traitées avec le composé d'essai ; et
la sélection d'un composé d'essai qui inhibe la phosphorylation du substrat d'ALK en comparaison d'un témoin négatif qui n'est pas traité avec le composé d'essai.

6. Procédé de criblage d'un médicament pour traiter :
(i) la maladie d'Alzheimer médiée par la protéine tau, dans laquelle la protéine tau est hyperphosphorylée ou agrégée ou dans laquelle il y a une neurotoxicité de la protéine tau, ou
(ii) une dégénérescence du lobe frontotemporel médiée par la protéine tau, dans laquelle la protéine tau est hyperphosphorylée ou agrégée ou dans laquelle il y a une neurotoxicité de la protéine tau,
comprenant :
le traitement d'un composé d'essai avec une solution réactionnelle incluant l'ALK, l'ATP et un substrat d'ALK ; et
la sélection d'un composé d'essai qui inhibe une interaction entre l'ALK et le substrat d'ALK en comparaison d'un témoin négatif qui n'est pas traité avec le composé d'essai.

7. Procédé *in vitro* pour cribler un médicament pour traiter :
(i) la maladie d'Alzheimer médiée par la protéine tau, dans laquelle la protéine tau est hyperphosphorylée ou agrégée ou dans laquelle il y a une neurotoxicité de la protéine tau, ou
(ii) une dégénérescence du lobe frontotemporel médiée par la protéine tau, dans laquelle la protéine tau est hyperphosphorylée ou agrégée ou dans laquelle il y a une neurotoxicité de la protéine tau,
comprenant :
le traitement de cellules exprimant l'ALK avec un composé d'essai et un substrat d'ALK ; et
la sélection d'un composé d'essai qui inhibe une interaction entre l'ALK et le substrat d'ALK en comparaison d'un témoin négatif qui n'est pas traité avec le composé d'essai.

8. Procédé de criblage d'un médicament pour traiter :
(i) la maladie d'Alzheimer médiée par la protéine tau, dans laquelle la protéine tau est hyperphosphorylée ou agrégée ou dans laquelle il y a une neurotoxicité de la protéine tau, ou
(ii) une dégénérescence du lobe frontotemporel médiée par la protéine tau, dans laquelle la protéine tau est hyperphosphorylée ou agrégée ou dans laquelle il y a une neurotoxicité de la protéine tau,
comprenant :
le traitement d'un composé d'essai avec une solution réactionnelle incluant de l'ALK et un ligand d'ALK ; et
la sélection d'un composé d'essai qui inhibe une interaction entre l'ALK et le ligand d'ALK en comparaison d'un témoin négatif qui n'est pas traité avec le composé d'essai.

9. Procédé *in vitro* pour cribler un médicament pour traiter :
(i) la maladie d'Alzheimer médiée par la protéine tau, dans laquelle la protéine tau est hyperphosphorylée ou agrégée ou dans laquelle il y a une neurotoxicité de la protéine tau, ou
(ii) une dégénérescence du lobe frontotemporel médiée par la protéine tau, dans laquelle la protéine tau est hyperphosphorylée ou agrégée ou dans laquelle il y a une neurotoxicité de la protéine tau,
comprenant :
le traitement de cellules exprimant l'ALK avec un ligand d'ALK et un composé d'essai ; et
la sélection d'un composé d'essai qui inhibe une interaction entre l'ALK et le ligand d'ALK en comparaison d'un témoin négatif qui n'est pas traité avec le composé d'essai.

10. Procédé *in vitro* pour cribler un médicament pour traiter :
(i) la maladie d'Alzheimer médiée par la protéine tau, dans laquelle la protéine tau est hyperphosphorylée ou agrégée ou dans laquelle il y a une neurotoxicité de la protéine tau, ou
(ii) une dégénérescence du lobe frontotemporel médiée par la protéine tau, dans laquelle la protéine tau est hyperphosphorylée ou agrégée ou dans laquelle il y a une neurotoxicité de la protéine tau,
comprenant :
le traitement de cellules exprimant l'ALK avec un composé d'essai ;
la mesure d'un changement dans un niveau de phosphorylation de kinase-5 dépendante de la cycline (CDK-5) ou d'une kinase régulée par un signal extracellulaire (ERK) dans des cellules traitées avec le composé d'essai ; et
la sélection d'un composé d'essai qui inhibe de manière significative la phosphorylation de la CDK-5 ou de l'ERK en comparaison d'un témoin négatif non traité avec le composé d'essai.

11. Procédé de criblage d'un médicament pour traiter :
(i) la maladie d'Alzheimer médiée par la protéine tau, dans laquelle la protéine tau est hyperphosphorylée ou agrégée ou dans laquelle il y a une neurotoxicité de la protéine tau, ou
(ii) une dégénérescence du lobe frontotemporel médiée par la protéine tau, dans laquelle la protéine tau est hyperphosphorylée ou agrégée ou dans laquelle il y a une neurotoxicité de la protéine tau,
comprenant:
le traitement d'une mouche à fruit transformée exprimant l'ALK et tau (Tau/dALK) avec un composé d'essai ;
l'observation d'un phénotype oculaire ou d'une dégénérescence neuronale en mesurant la forme d'une ommatidie ou un changement de l'épaisseur rétinienne de la mouche à fruit transformée avec le composé d'essai ; et
la sélection d'un composé d'essai qui inhibe de manière significative un changement de la forme d'une ommatidie ou la rupture d'une rétine en comparaison d'un témoin négatif non traité avec le composé d'essai.

12. Procédé de criblage d'un médicament pour traiter :
(i) la maladie d'Alzheimer médiée par la protéine tau, dans laquelle la protéine tau est hyperphosphorylée ou agrégée ou dans laquelle il y a une neurotoxicité de la protéine tau, ou
(ii) une dégénérescence du lobe frontotemporel médiée par la protéine tau, dans laquelle la protéine tau est hyperphosphorylée ou agrégée ou dans laquelle il y a une neurotoxicité de la protéine tau,
comprenant :
le traitement d'une protéine d'ALK avec un composé d'essai ;
l'observation du fait que la protéine d'ALK forme ou non un dimère ; et
la sélection d'un composé d'essai qui inhibe la formation de dimère de protéine d'ALK.

13. Procédé selon les revendications 8 ou 9, dans lequel le ligand d'ALK peut être la pléiotropie (PTN), la midkine (MK) ou le Jelly belly (Jeb).
